# EUROPÄISCHE PATENTSCHRIFT

(11) **EP 2 890 690 B1**
(45) Veröffentlichungstag und Bekanntmachung des Hinweises auf die Patenterteilung: **27.04.2016**
(21) Anmeldenummer: 13756046.2
(22) Anmeldetag: 22.08.2013
(51) Int. Cl.: C07D 317/36, C08G 18/08, C08G 18/79, C08G 18/80

(54) **BINDEMITTEL MIT CYCLISCHEN CARBONATSTRUKTUREN**
BINDER WITH CYCLIC CARBONATE STRUCTURES
LIANT AVEC STRUCTURES CARBONATES CYCLIQUES

(30) Priorität: 28.08.2012 EP 12182068
(43) Veröffentlichungstag der Anmeldung: 08.07.2015
(73) Patentinhaber: Covestro Deutschland AG, 51373 Leverkusen (DE)
(72) Erfinder: LAAS, Hans, Josef, 51519 Odenthal (DE); GRESZTA-FRANZ, Dorota, 42659 Solingen (DE); VEGA SANCHEZ, Berta, 50674 Köln (DE)
(74) Vertreter: Levpat
(86) Internationale Anmeldenummer: PCT/EP2013/067463
(87) Internationale Veröffentlichungsnummer: WO 2014/033045

(56) Entgegenhaltungen:
- WO-A1-2008/125419

## Beschreibung

Die Erfindung betrifft ein Verfahren zur Herstellung von cyclische Carbonatstrukturen enthaltenden Polyurethanen, die nach diesem Verfahren erhältlichen cyclische Carbonatstrukturen enthaltenden Polyurethane, deren Verwendung als Bindemittel in vernetzbaren Lack-, Dichtstoff- oder Klebstoffrohstoffen, sowie die die erfindungsgemäßen Strukturen enthaltenden vernetzbaren Bindemittel.

Polyurethane, die cyclische Carbonatgruppen tragen, sind als Bindemittel für Klebstoffe, Dichtstoffe oder Beschichtungen seit längerem bekannt und bereits mehrfach beschrieben.

Eine sehr einfache, häufig genutzte Methode zur Herstellung solcher Polyurethane besteht in der Umsetzung niedermolekularer hydroxyfunktioneller cyclischer Carbonate mit Polyisocyanaten oder isocyanatfunktionellen Prepolymeren.

Beispielsweise beschreibt die WO 2006/010408 isocyanatgruppenfreie Umsetzungsprodukte linearer Polyurethanpräpolymere auf Basis von Diphenylmethandiisocyanat (MDI) mit 4-(Hydroxymethyl)-1,3-dioxolan-2-on (Glycerincarbonat), die sich mit Verbindungen, die mindestens zwei primäre oder sekundäre Aminogruppen tragen, bereits bei Raumtemperatur vernetzen lassen. Solche Zweikomponenten-Bindemittel finden als Kleb- und Dichtstoffe, insbesondere als Kaschierklebstoff für Verbundfolien, Verwendung.

Mit hydroxyfunktionellen cyclischen Carbonaten blockierte Polyisocyanate, beispielsweise Umsetzungsprodukte von Polyisocyanuratpolyisocyanaten des 1,6-Diisocyanatohexan (Hexamethylendiisocyanat, HDI) mit Glycerincarbonat, sind Gegenstand der WO 2008/125419. In speziellen Acetalen, wie insbesondere 1,1,2,2-Tetramethoxyethan, gelöst, härten solche Glycerincarbonat/Polyisocyanat-Addukte mit Polyetherpolyaminen oder Polyamidaminen ebenfalls bereits bei Raumtemperatur zu Lackfilmen hoher optischer Qualität aus.

Verbindungen, die cyclische Carbonatgruppen tragen, lassen sich auch mit hydroxyfunktionellen Reaktionspartnern kombinieren. Während die aminische Härtung der cyclischen Carbonatgruppen bereits bei niedrigen Temperaturen ausreichend schnell verläuft, erfolgt eine Vernetzung in diesem Fall allerdings nur bei erhöhten Temperaturen, in der Regel unter Einbrennbedingungen, und in Gegenwart spezieller Katalysatoren.

EP-A 0 911 352 beschreibt beispielsweise bei Raumtemperatur lagerstabile Einkomponentensysteme, bestehend aus Polyolen, Umsetzungsprodukten von Polyisocyanuratpolyisocyanaten des HDI und/oder des 1-Isocyanato-3,3,5-trimethyl-5-isocyanatomethylcyclohexans (Isophorondiisocyanat, IPDI) mit 5-(Hydroxymethyl)-5-ethyl-1,3-dioxan-2-on (TMP-Carbonat) als Vernetzerkomponenten und speziellen Metallcarboxylaten als Aushärtekatalysatoren, die ohne Abspaltung flüchtiger Verbindungen bei erhöhten Temperaturen zu harten lösemittelbeständigen Beschichtungen eingebrannt werden können.

Die Herstellung 1,3-Dioxan-2-on-Gruppen enthaltender Oligourethane aus Diisocyanaten oder Polyisocyanaten unter Verwendung von TMP-Carbonat und ihre Verwendung als Vernetzer für Polyole in thermisch härtbaren Beschichtungssystemen ist auch aus EP-A 0 703 230 bekannt.

Bei den heute bekannten, auf hydroxyfunktionellen cyclischen Carbonaten beruhenden Reaktivsystemen handelt es sich somit entweder um Umsetzungsprodukte derartiger Bausteine mit vergleichsweise niedermolekularen oligomeren Polyisocyanaten oder mit höhermolekularen Isocyanatpräpolymeren, die in der Regel aber linear aufgebaut sind. Hochmolekulare und gleichzeitig hochfunktionelle Polyisocyanatkomponenten wurden als Reaktionspartner für hydroxyfunktionelle Cyclocarbonate bisher nicht beschrieben. Gerade polymere Polycyclocarbonatpolyurethane mit hohem Molekulargewicht sollten aber als Bindemittelkomponenten von besonderem Interesse sein, da sie z. B. in Kombination mit den kommerziell gut verfügbaren niedermolekularen Polyaminen, wie sie beispielsweise als Vernetzer für Epoxysysteme dienen, zu hochvernetzten besonders beständigen Polyurethanen abreagieren sollten.

Die Hauptursache für das bisherige Fehlen hochfunktioneller polymerer Polycyclocarbonatpolyurethane liegt darin, dass monomerarme NCO-Prepolymere hochmolekularer verzweigter Polyole aufgrund der während der Prepolymerisierungsreaktion mit Diisocyanaten unvermeidlich einsetzenden Vernetzungsreaktionen und dem damit verbundenen Molekulargewichtsaufbau gelieren oder zumindest extrem hohe Viskositäten aufweisen, die sie als Bausteine für eine Umsetzung mit hydroxyfunktionellen cyclischen Carbonaten unbrauchbar machen.

Es bestand daher ein Bedarf an neuen Polyurethanen mit cyclischen Carbonatstrukturen, die aus beliebigen, d. h. auch hochverzweigten polymeren Polyolen auf einfache Weise sicher und reproduzierbar herstellbar sind.

Aufgabe der vorliegenden Erfindung war es daher, ein Verfahren zur Herstellung niedrigviskoser cyclische Carbonatstrukturen tragender Polyurethane aus beliebigen Polyolen zur Verfügung zu stellen, das auf Aufbaukomponenten basiert, die auch in technischem Maßstab leicht und reproduzierbar zugänglich sind. Die nach diesem Verfahren erhältlichen Produkte sollten sich für sämtliche Anwendungsgebiete cyclische Carbonatstrukturen enthaltender Polyurethane, insbesondere als Bindemittel für Klebstoffe, Dichtstoffe oder Beschichtungen eignen.

Diese Aufgabe wird erfindungsgemäß gelöst durch ein Verfahren zur Herstellung von cyclische Carbonatstrukturen enthaltenden Polyurethanen, durch Umsetzung von wenigstens
A) einer Zusammensetzung enthaltend
   a) eine oder mehrere Isocyanatgruppen und cyclische Carbonatstrukturen enthaltende Verbindungen der allgemeinen Formel (I) 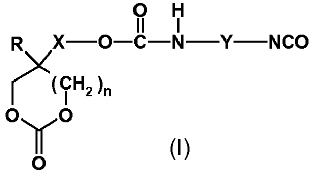 und
   b) ≤ 1 Gew.-%, bezogen auf die Gesamtmasse der Zusammensetzung A) eines oder mehrerer monomerer Diisocyanate mit aliphatisch, cycloaliphatisch, araliphatisch und/oder aromatisch gebundenen Isocyanatgruppen der allgemeinen Formel (II),

      **OCN-Y-NCO** (II)

      wobei
      - R: für Wasserstoff oder einen gesättigten oder ungesättigten, linearen oder verzweigten, aliphatischen Rest mit 1 bis 7 Kohlenstoffatomen steht,
      - X: für einen linearen oder verzweigten organischen Rest mit 1 bis 36 Kohlenstoffatomen steht, der gegebenenfalls Ether-, Ester- und/oder Carbonatgruppen enthalten kann,
      - n: für 0 oder 1 steht und
      - Y: für einen linearen oder verzweigten, aliphatischen oder cycloaliphatischen Rest mit 4 bis 18 Kohlenstoffatomen oder einen gegebenenfalls substituierten aromatischen oder araliphatischen Rest mit 6 bis 18 Kohlenstoffatomen steht,
      mit
B) einem mindestens difunktionellen Polyol mit einem zahlenmittleren Molekulargewicht Mₙ von 62 bis 22000 g/mol, bevorzugt 90 bis 12000 g/mol
   unter Einhaltung eines Äquivalentverhältnisses von Isocyanatgruppen zu Hydroxylgruppen von 0,3 : 1 bis 1,2 : 1.

Die im gesamten Dokument genannten zahlenmittleren Molekulargewichte Mₙ der polymeren Polyole werden mittels Gelpermeationschromatographie (GPC) wie folgt bestimmt: Die Kalibrierung erfolgt mit Polystyrol-Standards mit Molekulargewichten von Mp 1.000.000 bis 162. Als Eluent wird Tetrahydrofuran p.A. verwendet. Die folgenden Parameter werden bei der Doppelmessung eingehalten: Messung bei Raumtemperatur; Entgasung: Online - Degasser; Durchfluß: 1 ml/Min; Analysenzeit: 45 Minuten; Detektoren: Refraktometer und UV-Detektor; Injektionsvolumen: 100 µl - 200 µl. Die Berechnung von Molmassenmittelwerte Mₙ erfolgt softwaregestützt. Basislinienpunkte und Auswertegrenzen werden entsprechend der DIN 55672 Teil 1 festgelegt. Im Falle niedermolekularer Polyole mit definierter Struktur gilt das aus der Summenformel errechenbare Molekulargewicht.

Das erfindungsgemäße Verfahren beruht auf der überraschenden Beobachtung, dass sich durch Reaktion üblicher hydroxyfunktioneller cyclischer Carbonate mit überschüssigen Mengen monomerer Diisocyanate und nachfolgende Abtrennung der nicht umgesetzten Monomeren Verbindungen erhalten lassen, die gleichzeitig eine Isocyanatgruppe und eine cyclische Carbonatgruppe aufweisen und mit denen sich auch hochverzweigte polymere Polyole problemlos zu kristallisationsstabilen Polyurethanen mit Cyclocarbonat-Endgruppen umsetzen lassen.

Der Bestandteil b) der Zusammensetzung A) hat einen Anteil von ≤ 1 Gew.-%, bevorzugt ≤ 0,5 Gew.-% und besonders bevorzugt ≤ 0,3 Gew.-% an der Gesamtmasse der Zusammensetzung A).

In einer bevorzugten Ausführungsform des erfindungsgemäßen Verfahrens enthält die Zusammensetzung A) zusätzlich Verbindungen (c) der allgemeinen Formel (III) 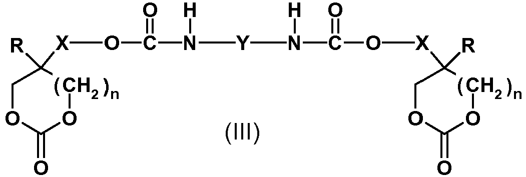 in einer Menge von 0,5 bis 12 Gew.-%, bevorzugt 0,5 bis 10 Gew.-%, bezogen auf die Gesamtmasse der Komponenten a) und c), wobei
- R: für Wasserstoff oder einen gesättigten oder ungesättigten, linearen oder verzweigten, aliphatischen Rest mit 1 bis 7 Kohlenstoffatomen steht,
- X: für einen linearen oder verzweigten organischen Rest mit 1 bis 36 Kohlenstoffatomen steht, der gegebenenfalls Ether-, Ester- und/oder Carbonatgruppen enthalten kann,
- n: für 0 oder 1 steht und
- Y: für einen linearen oder verzweigten, aliphatischen oder cycloaliphatischen Rest mit 4 bis 18 Kohlenstoffatomen oder einen gegebenenfalls substituierten aromatischen oder araliphatischen Rest mit 6 bis 18 Kohlenstoffatomen steht,

In einer weiteren bevorzugten Ausführungsform des erfindungsgemäßen Verfahrens enthält die Zusammensetzung A)
a) ≥ 88 Gew.-%, bevorzugt ≥ 90 Gew.-% und besonders bevorzugt ≥ 92 Gew.-% an Verbindungen der allgemeinen Formel (I),
b) ≤ 1 Gew.-%, bevorzugt ≤ 0,5 Gew.-%, besonders bevorzugt ≤ 0,3 Gew.-% eines oder mehrerer monomerer Diisocyanate mit aliphatisch, cycloaliphatisch, araliphatisch und/oder aromatisch gebundenen Isocyanatgruppen der allgemeinen Formel (II) und
c) ≤ 12 Gew.-%, bevorzugt ≤ 10 Gew.-% und besonders bevorzugt ≤ 8 Gew.-% an Verbindungen der allgemeinen Formel (III),
wobei sich der Anteil an a) und c) jeweils auf die Gesamtmasse der Verbindungen a) und c) bezieht, der Anteil an b) sich auf die Gesamtmasse der Zusammensetzung A) bezieht,
und wobei
- R: für Wasserstoff oder einen gesättigten oder ungesättigten, linearen oder verzweigten, aliphatischen Rest mit 1 bis 7 Kohlenstoffatomen steht,
- X: für einen linearen oder verzweigten organischen Rest mit 1 bis 36 Kohlenstoffatomen steht, der gegebenenfalls Ether-, Ester- und/oder Carbonatgruppen enthalten kann,
- n: für 0 oder 1 steht und
- Y: für einen linearen oder verzweigten, aliphatischen oder cycloaliphatischen Rest mit 4 bis 18 Kohlenstoffatomen oder einen gegebenenfalls substituierten aromatischen oder araliphatischen Rest mit 6 bis 18 Kohlenstoffatomen steht.

Die Zusammensetzung A) wird dabei bevorzugt durch Umsetzung von monomeren Diisocyanaten mit aliphatisch, cycloaliphatisch, araliphatisch und/oder aromatisch gebundenen Isocyanatgruppen i) mit hydroxyfunktionellen cyclischen Carbonaten ii) in einem einem Äquivalentverhältnis von Isocyanatgruppen zu Hydroxylgruppen von mindestens 8 : 1, bevorzugt mindestens 10 : 1 und besonders bevorzugt mindestens 12 : 1 erhalten.

Isocyanatgruppen und cyclische Carbonatstrukturen enthaltende Verbindungen, die unter Verwendung eines geringeren Düsocyanatüberschusses hergestellt wurden und wie sie beispielsweise in der EP-A 0 703 230 und EP-A 0 328 150 beschrieben sind, liefern im Gegensatz zu den erfindungsgemäß eingesetzten Zusammensetzungen A) bei analoger Umsetzung mit Polyolen aufgrund ihres zu hohen Anteils an 2:1-Bisaddukt aus hydroxyfunktionellem cyclischen Carbonat und Diisocyanat immer trübe Polycyclocarbonatpolyurethane, die als Bindemittel für Lacke und Beschichtungen unbrauchbar sind.

Als Ausgangsverbindungen i) für die Herstellung der Zusammensetzung A) sind beliebige Diisocyanate mit aliphatisch, cycloaliphatisch, araliphatisch und/oder aromatisch gebundenen Isocyanatgruppen geeignet, die nach beliebigen Verfahren, z. B. durch Phosgenierung oder auf phosgenfreiem Weg, beispielsweise durch Urethanspaltung, hergestellt werden können.

Geeignete Diisocyanate sind beispielsweise solche der allgemeinen Formel (II)

**OCN-Y-NCO** (II)

in welcher Y für einen linearen oder verzweigten, aliphatischen oder cycloaliphatischen Rest mit 4 bis 18 Kohlenstoffatomen oder einen gegebenenfalls substituierten aromatischen oder araliphatischen Rest mit 6 bis 18 Kohlenstoffatomen steht, wie z. B. 1,4-Diisocyanatobutan, 1,6-Diisocyanatohexan (HDI), 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,4-Diisocyanato-3,3,5-trimethylcyclohexan, 1,3-Diisocyanato-2-methylcyclohexan, 1,3-Diisocyanato-4-methylcyclohexan, 1-Isocyanato-3,3,5-trimethyl-5-isocyanatomethylcyclohexan (Isophorondiisocyanat; IPDI), 1-Isocyanato-1-methyl-4(3)-isocyanatomethylcyclohexan, 2,4'- und 4,4'-Diisocyanatodicyclohexylmethan (H₁₂-MDI), 1,3- und 1,4-Bis(isocyanatomethyl)cyclohexan, 4,4'-Diisocyanato-3,3'-dimethyldicyclohexylmethan, 4,4'-Diisocyanato-3,3',5,5'-tetramethyldicyclohexylmethan, 4,4'-Diisocyanato1,1'-bi(cyclohexyl), 4,4'-Diisocyanato-3,3'-dimethyl-1,1'-bi(cyclohexyl), 4,4'-Diisocyanato-2,2',5,5'-tetra-methyl-1,1'-bi(cyclohexyl), 1,8-DÜsocyanato-p-menthan, 1,3-Diisocyanatoadamantan, 1,3-Dimethyl-5,7-diisocyanatoadamantan, 1,3- und 1,4-Bis-(isocyanatomethyl)benzol (XDI), 1,3- und 1,4-Bis(1-isocyanato-1-methylethyl)-benzol (TMXDI), Bis(4-(1-isocyanato-1-methylethyl)phenyl)-carbonat, 1,3- und 1,4-Phenylendiisocyanat, 2,4- und 2,6-Toluylendiisocyanat (TDI) sowie beliebige Gemische dieser Isomeren, Diphenylmethan-2,4'- und/oder -4,4'-diisocyanat (MDI) und Naphthylen-1,5-diisocyanat (NDI) sowie beliebige Gemische solcher Diisocyanate.

Weitere ebenfalls geeignete Diisocyanate finden sich darüber hinaus beispielsweise in Justus Liebigs Annalen der Chemie Band 562 (1949) S. 75 - 136.

Als Ausgangskomponente i) bevorzugt sind Diisocyanate der allgemeinen Formel (II), in welcher Y für einen linearen oder verzweigten, aliphatischen oder cycloaliphatischen Rest mit 6 bis 13 Kohlenstoffatomen steht.

Besonders bevorzugte Ausgangskomponenten i) sind 1,6-Diisocyanatohexan, 1-Isocyanato-3,3,5-trimethyl-5-isocyanatomethylcyclohexan, 2,4'- und/oder 4,4'-Diisocyanatodicyclohexylmethan oder deren Gemische.

Als Ausgangskomponenten ii) zur Herstellung der Zusammensetzung A) eignen sich hydroxyfunktionelle cyclische Carbonate der allgemeinen Formel (IV) oder deren Gemische, 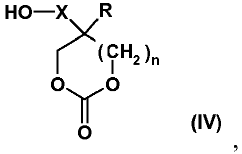 in welcher
- R: für Wasserstoff oder einen gesättigten oder ungesättigten, linearen oder verzweigten, aliphatischen Rest mit 1 bis 7 Kohlenstoffatomen steht,
- X: für einen linearen oder verzweigten organischen Rest mit 1 bis 36 Kohlenstoffatomen steht, der gegebenenfalls Ether-, Ester- und/oder Carbonatgruppen enthalten kann, und
- n: für 0 oder 1 steht.

Geeignete Ausgangskomponenten ii) sind beispielsweise einfache hydroxyfunktionelle Cyclocarbonate, wie z. B. 4-(Hydroxymethyl)-1,3-dioxolan-2-on (Glycerincarbonat), 5-(Hydroxymethyl)-5-methyl-1,3-dioxan-2-on und/oder 5-(Hydroxymethyl)-5-ethyl-1,3-dioxan-2-on (TMP-Carbonat).

Geeignete Ausgangskomponenten ii) sind daneben auch die durch Umsetzung dieser einfachen hydroxyfunktionellen cyclischen Carbonate mit Alkylenoxiden, wie z. B. Ethylenoxid und/oder Propylenoxid, Lactonen, wie z. B. ß-Propiolacton, γ-Butyrolacton, δ-Valerolacton, ε-Caprolacton, 3,5,5-und 3,3,5-Trimethylcaprolacton, und/oder cyclischen Carbonaten, wie z. B. 1,3-Dioxan-2-on (Trimethylencarbonat) und 5,5-Dimethyl-1,3-dioxan-2-on (Neopentylglykolcarbonat), nach bekannten Methoden erhältlichen, cyclische Carbonatstrukturen aufweisenden Polyetheralkohole, Polyesteralkohole und/oder Polycarbonatalkohole mit zahlenmittleren Molekulargewichten Mₙ von bis zu 600 g/mol, vorzugsweise bis zu 500 g/mol, besonders bevorzugt bis zu 400 g/mol.

Die Herstellung der Ausgangsverbindungen ii) ist nicht Gegenstand der vorliegenden Anmeldung. Sie können nach bekannten Verfahren beispielsweise unter Verwendung fossiler Rohstoffe oder auch aus nachwachsenden Rohstoffen, wie z. B. aus biogenem Glycerin, wie es beispielsweise als Koppelprodukt der Biodieselherstellung anfällt, erhalten werden.

Als mögliche Herstellverfahren für die obengenannten einfachen hydroxyfunktionellen cyclischen Carbonate seien hier beispielhaft genannt Umsetzungen von Glycerin oder Trimethylolpropan mit Ethylencarbonat, Dialkyl- oder Diarylcarbonaten, wie z. B. Dimethyl- oder Diphenylcarbonat, unter Umesterungsbedingungen (siehe z. B. EP-A 1 963 301, EP-A 0 739 888, DE-A 196 25 265 und WO 2011/159219). Daneben kann Glycerincarbonat auch durch direkte Umsetzung von Glycidol mit Kohlendioxid in Gegenwart geeigneter Katalysatoren gewonnen werden
(siehe z. B. EP-A 0 229 622).

Verfahren zur Herstellung der als Ausgangsverbindungen ii) ebenfalls geeigneten Verbindungen, die zusätzlich mindestens eine Polyether-, Polyester- und/oder Polycarbonatgruppe enthalten sind ebenfalls bereits bekannt.

So liefert beispielsweise die Umsetzung einfacher hydroxyfunktioneller cyclischer Carbonate mit Alkylenoxiden, insbesondere mit Propylenoxid, nach den üblichen Verfahren zur Synthese von Polyethern (siehe z. B. N. Adam et al.: "Polyurethanes", Ullmann's Encyclopedia of Industrial Chemistry, Electronic Release, 7th ed., chap. 3.2.1, Wiley-VCH, Weinheim 2005) in Gegenwart von KOH oder insbesondere nach dem IMPACT-Prozess unter Verwendung von Doppelmetallcyanid-Katalysatoren (DMC) endständig cyclische Carbonatstrukturen aufweisende Polyetheralkohole, die als Ausgangsverbindung ii) geeignet sind.

Bevorzugte mindestens eine Ethergruppe aufweisende Ausgangsverbindungen ii) sind solche des obengenannten Molekulargewichtsbereiches auf Basis von Glycerincarbonat und Propylenoxid.

Geeignete Ausgangsverbindungen ii), die neben einer cyclischen Carbonatgruppe mindestens eine Estergruppe enthalten, lassen sich in an sich bekannter Weise aus Lactonen und den oben beschriebenen einfachen hydroxyfunktionellen cyclischen Carbonaten als Startermolekülen unter Ringöffnung herstellen. Geeignete Lactone zur Herstellung dieser Estergruppen enthaltenden Ausgangsverbindungen ii) sind beispielsweise ß-Propiolacton, γ-Butyrolacton, δ-Valerolacton, ε-Caprolacton, 3,5,5- und 3,3,5-Trimethylcaprolacton oder beliebigen Gemische solcher Lactone.

Bevorzugte mindestens eine Estergruppe aufweisende Ausgangsverbindungen ii) sind solche des obengenannten Molekulargewichtsbereiches auf Basis von Glycerincarbonat und ε-Caprolacton. Als Ausgangsverbindungen ii) ebenfalls geeignet sind schließlich auch Verbindungen, die neben einer cyclischen Carbonatgruppe mindestens eine weitere Carbonatgruppe enthalten. Derartige Verbindungen sind ebenfalls bereits bekannt und beispielsweise nach dem in Beispiel 1), Step (A) der WO 03/016298 beschriebenen Verfahren durch Umsetzung der oben beschriebenen einfachen hydroxyfunktionellen cyclischen Carbonate mit Trimethylencarbonat und/oder Neopentylglykolcarbonat erhältlich.

Bevorzugte mindestens eine Carbonatgruppe aufweisende Ausgangsverbindungen ii) sind solche des obengenannten Molekulargewichtsbereiches auf Basis von Glycerincarbonat und Neopentylglykolcarbonat.

Im Allgemeinen erfolgt die Herstellung der vorstehend beschriebenen Ausgangsverbindungen ii), die neben einer cyclischen Carbonatgruppe mindestens eine Estergruppe und/oder mindestens eine weitere Carbonatgruppe enthalten, durch ringöffnende Polymerisation in Gegenwart von Katalysatoren wie beispielsweise Lewis- oder Brönstedt-Säuren, organischen Zinn- oder Titanverbindungen bei Temperaturen von 20 bis 200°C, vorzugsweise 50 bis 160°C.

Des weiteren bevorzugte Ausgangskomponenten ii) sind hydroxyfunktionelle cyclische Carbonate der allgemeinen Formel (IV) oder deren Gemische, in welcher
- R: für Wasserstoff oder einen gesättigten linearen aliphatischen Rest mit 1 oder 2 Kohlenstoffatomen steht,
- X: für einen linearen oder verzweigten organischen Rest mit 1 bis 18 Kohlenstoffatomen steht, der gegebenenfalls Ether-, Ester- und/oder Carbonatgruppen enthalten kann, und
- n: für 0 oder 1 steht.

Besonders bevorzugte Ausgangskomponenten ii) sind hydroxyfunktionelle cyclische Carbonate der allgemeinen Formel (IV) oder deren Gemische, in welcher
- R: für Wasserstoff oder einen gesättigten linearen aliphatischen Rest mit 1 oder 2 Kohlenstoffatomen steht,
- X: für eine Methylengruppe (-CH₂-) und
- n: für 0 oder 1 steht.

Ganz besonders bevorzugte Ausgangskomponente ii) ist Glycerincarbonat.

Die hydroxyfunktionellen cyclischen Carbonate ii) können sowohl einzeln als auch in Form beliebiger Gemische untereinander eingesetzt werden.

Zur Herstellung der Zusammensetzung A) werden die Diisocyanate i) bevorzugt mit mindestens einem hydroxyfunktionellen cyclischen Carbonat ii) bei Temperaturen von 20 bis 200 °C, vorzugsweise 40 bis 160 °C umgesetzt.

Dabei wird Komponente i) mit Komponente ii) bevorzugt in einem Äquivalentverhältnis von Isocyanatgruppen zu Hydroxylgruppen von mindestens 8:1, bevorzugt mindestens 10 : 1 und besonders bevorzugt mindestens 12:1 umgesetzt. Ebenfalls bevorzugt wird die Komponente i) mit Komponente ii) in einem Äquivalentverhältnis von Isocyanatgruppen zu Hydroxylgruppen von höchstens 40 : 1, bevorzugt von höchstens 30:1 umgesetzt.

Die Umsetzung der Ausgangskomponenten i) und ii) kann in Lösung oder lösemittelfrei in Substanz, bevorzugt jedoch lösemittelfrei ausgeführt werden.

Die Reaktion kann unkatalysiert durchgeführt werden. Gegebenenfalls können zur Beschleunigung der Umsetzung aber auch übliche aus der Polyurethanchemie bekannte Katalysatoren mitverwendet werden. Beispielhaft seien hier genannt tert. Amine, wie z. B. Triethylamin, Tributylamin, Dimethylbenzylamin, Diethylbenzylamin, Pyridin, Methylpyridin, Dicyclohexylmethylamin, Dimethylcyclohexylamin, N,N,N',N'-Tetramethyldiaminodiethylether, Bis-(dimethylaminopropyl)-harnstoff, N-Methyl- bzw. N-Ethylmorpholin, N-Cocomorpholin, N-Cyclohexylmorpholin, N,N,N',N'-Tetramethylethylendiamin, N,N,N',N'-Tetramethyl-1,3-butandiamin, N,N,N',N'-Tetramethyl-1,6-hexandiamin, Pentamethyldiethylentriamin, N-Methylpiperidin, N-Dimethylaminoethylpiperidin, N,N'-Dimethylpiperazin, N-Methyl-N'-dimethylaminopiperazin, 1,2-Dimethylimidazol, 2-Methylimidazol, N,N-Dimethylimidazol-β-phenylethylamin, 1,4-Diazabicyclo-(2,2,2)-octan (DABCO) und Bis-(N,N-dimethylaminoethyl)adipat, Amidine, wie z. B. 1,5-Diazabicyclo[4.3.0]nonen (DBN), 1,8-Diazabicyclo(5.4.0)undecen-7 (DBU) und 2,3-Dimethyl-3,4,5,6-tetrahydropyrimidin, Alkanolaminverbindungen, wie z. B. Triethanolamin, Triisopropanolamin, N-Methyl-diethanolamin, N-Ethyl-diethanolamin, Dimethylaminoethanol und 2-(N,N-Dimethylaminoethoxy)ethanol, N,N',N"-Tris-(dialkylaminoalkyl)hexahydrotriazine, wie z. B. N,N',N"-Tris-(dimethylaminopropyl)-s-hexahydrotriazin, Bis(dimethylaminoethyl)ether sowie Metallsalze, wie z. B. anorganische und/oder organische Verbindungen des Eisens, Bleis, Wismuths, Zinks, und/oder Zinns in üblichen Oxidationsstufen des Metalls, beispielsweise Eisen(II)-chlorid, Eisen(III)-chlorid, Wismut(III)- Wismut(III)-2-ethylhexanoat, Wis-mut(III)-octoat, Wismut(III)-neodecanoat, Zinkchlorid, Zink-2-ethylcaproat, Zinn(II)-octoat, Zinn(II)-ethylcaproat, Zinn(II)-palmitat, Dibutylzinn(IV)-dilaurat (DBTL), Dibutylzinn(IV)-dichlorid oder Bleioctoat.

Bevorzugt einzusetzende Katalysatoren sind tertiäre Amine, Zinn-, Zink und Wismutverbindungen der genannten Art.

Die beispielhaft genannten Katalysatoren können bei der Herstellung der Zusammensetzung A) einzeln oder in Form beliebiger Mischungen untereinander eingesetzt werden und kommen dabei, falls überhaupt, in Mengen von 0,001 bis 1,0 Gew.-%, bevorzugt 0,005 bis 0,5 Gew.-%, berechnet als Gesamtmenge an eingesetzten Katalysatoren bezogen auf die Gesamtmenge der verwendeten Ausgangsverbindungen, zum Einsatz.

Der Verlauf der Umsetzung kann durch z. B. titrimetrische Bestimmung des NCO-Gehaltes verfolgt werden. Nach Erreichen des angestrebten NCO-Gehaltes, im allgemeinen nach vollständiger Urethanisierung, wird die Reaktion abgebrochen.

In einer bevorzugten Ausführungsform wird nach der Umsetzung der Komponenten i) und ii) ein nicht umgesetzter Überschusses an monomeren Diisocyanaten i) bis auf einen Restgehalt von ≤ 1 Gew.-%, vorzugsweise von ≤ 0,5 Gew.-%, besonders bevorzugt von ≤ 0,3 Gew.-%, bezogen auf die Gesamtmasse des Reaktionsproduktes vom Reaktionsprodukt abgetrennt.

Das Reaktionsgemisch wird dabei vorzugsweise durch Dünnschichtdestillation im Vakuum, beispielsweise bei einem Druck von unter 1,0 mbar, vorzugsweise unter 0,5 mbar, besonders bevorzugt unter 0,2 mbar, unter möglichst schonenden Bedingungen, beispielsweise bei einer Temperatur von 100 bis 200 °C, vorzugsweise von 120 bis 180 °C, von überschüssigen monomeren Diisocyanaten i) befreit.

Die anfallenden Destillate können problemlos zur erneuten Umsetzung mit geeigneten Ausgangsverbindungen ii) verwendet werden.

In einer weiteren, jedoch weniger bevorzugten Ausführungsform werden die monomeren Diisocyanate durch Extraktion mit geeigneten gegenüber Isocyanatgruppen inerten Lösungsmitteln, beispielsweise aliphatischen oder cycloaliphatischen Kohlenwasserstoffen wie Pentan, Hexan, Heptan, Cyclopentan oder Cyclohexan vom gebildeten erfindungsgemäßen Verfahrensprodukt abgetrennt.

Unabhängig von der Art der Aufarbeitung erhält man klare, praktisch farblose Zusammensetzungen A), die in Abhängigkeit vom gewählten Ausgangsdiisocyanat niedrig- bis hochviskose Flüssigkeiten oder feste Stoffe darstellen und NCO-Gehalte von 4,5 bis 14,8 Gew.-%, vorzugsweise 5,0 bis 14,8 Gew.-%, besonders bevorzugt 9,0 bis 14,5 Gew.-% sowie Restgehalte an monomeren Diisocyanaten von weniger als 1,0 Gew.-%, vorzugsweise von weniger als 0,5 Gew.-%, besonders bevorzugt von weniger als 0,3 Gew.-%, bezogen auf die Gesamtmasse der Zusammensetzung A), aufweisen.

Als Bestandteil b) der Zusammensetzung A) können folglich alle zur Herstellung der Zusammensetzung A) eingesetzten monomeren Diisocyanate i) auftreten. Für b) gelten auch die für die Komponente ii) oben angegebenen bevorzugten Ausführungsformen.

Bevorzugt gilt für die Formeln (I) und (II) der Komponenten a) und b) oder für die Formeln (I), (II) und (III) der Komponenten a), b) und c), dass
- Y: für einen linearen oder verzweigten, aliphatischen oder cycloaliphatischen Rest mit 6 bis 13 Kohlenstoffatomen steht.

Des Weiteren gilt für die Formel (I) der Komponente a) oder die Formeln (I) und (III) der Komponenten a) und c) bevorzugt, dass
- R: für Wasserstoff oder einen gesättigten linearen aliphatischen Rest mit 1 oder 2 Kohlenstoffatomen steht,
- X: für einen linearen oder verzweigten organischen Rest mit 1 bis 18 Kohlenstoffatomen steht, der gegebenenfalls Ether-, Ester- und/oder Carbonatgruppen enthalten kann, und
- n: für 0 oder 1 steht.

Besonders bevorzugt gilt für die Formel (I) der Komponente a) oder die Formeln (I) und (III) der Komponenten a) und c), dass
- R: für Wasserstoff oder einen gesättigten linearen aliphatischen Rest mit 1 oder 2 Kohlenstoffatomen steht,
- X: für eine Methylengruppe (-CH₂-) steht und
- n: für 0 oder 1 steht.

Bei den beim erfindungsgemäßen Verfahren eingesetzten Polyolkomponenten B) handelt es um beliebige mindestens difunktionellen Polyole mit einem zahlenmittleren Molekulargewicht Mₙ von 62 bis 22000 g/mol, bevorzugt 90 bis 18000 g/mol, besonders bevorzugt 90 bis 12000 g/mol.

Vorzugsweise weisen die beim erfindungsgemäßen Verfahren eingesetzten Polyolkomponenten B) eine mittlere Funktionalität von 2 bis 6 und besonders bevorzugt eine mittlere Funktionalität von 2 bis 4 auf.

Auch beliebige Gemische der genannten Polyole können eingesetzt werden.

In einer bevorzugten Ausführungsform der Erfindung kommen als Komponente B) einfache mehrwertige Alkohole, Ether- oder Esteralkohole und/oder polymere Polyole zum Einsatz. wobei die polymeren Polyole ein zahlenmittleren Molekulargewicht Mₙ von 200 bis 22000 g/mol, bevorzugt 250 bis 18000 g/mol besonders bevorzugt 250 bis 12000 g/mol aufweisen.

Nach einer speziellen Ausführungsform des erfindungsgemäßen Verfahrens werden als Polyolkomponente B) mehrwertige Alkohole und/oder Ether- oder Esteralkohole eingesetzt, die 2 bis 14 Kohlenstoffatome, bevorzugt 4 bis 10 Kohlenstoffatome enthalten.

Geeignete Polyole B) sind beispielsweise einfache mehrwertige Alkohole mit 2 bis 14, vorzugsweise 4 bis 10 Kohlenstoffatomen, wie z. B. 1,2-Ethandiol, 1,2- und 1,3-Propandiol, die isomeren Butandiole, Pentandiole, Hexandiole, Heptandiole und Octandiole, 1,10-Decandiol, 1,12-Dodecandiol, 1,2- und 1,4-Cyclohexandiol, 1,4-Cyclohexandimethanol, 1,4-Bis(2-hydroxyethoxy)-benzol, 2,2-Bis-(4-hydroxyphenyl)-propan (Bisphenol A), 2,2-Bis-(4-hydroxycyclohexyl)-propan (Perhydrobisphenol), 1,2,3-Propantriol, 1,2,4-Butantriol, 1,1,1-Trimethylolethan, 1,2,6-Hexantriol, 1,1,1-Trimethylolpropan (TMP), Bis-(2-hydroxyethyl)-hydrochinon, 1,2,4- und 1,3,5-Trihydroxycyclohexan, 1,3,5-Tris(2-hydroxyethyl)-isocyanurat, 3(4),8(9)-Bis-(hydroxymethyl)-tricyclo-[5.2.1.0^{2,6}]decane, Di-Trimethylolpropan, 2,2-Bis(hydroxymethyl)-1,3-propandiol (Pentaerythrit), 2,2,6,6-Tetrakis(hydroxymethyl)-4-oxa-heptan-1,7-diol (Dipentaerythrit), Mannitol oder Sorbitol, niedermolekulare Etheralkohole, wie z. B. Diethylenglykol, Triethylenglykol, Tetraethylenglykol, Dipropylenglykol oder Dibutylenglykol oder niedermolekulare Esteralkohole, wie z. B. Hydroxypivalinsäureneopentylglykolester.

Nach einer anderen speziellen Ausführungsform des erfindungsgemäßen Verfahrens werden als Polyolkomponente B) die üblichen aus der Polyurethanchemie bekannten polymeren Polyetherpolyole, Polyesterpolyole, Polycarbonatpolyole und/oder Polyacrylatpolyole, die üblicherweise ein zahlenmittleres Molekulargewicht Mₙ von 200 bis 22000 g/mol, vorzugsweise von 250 bis 18000 g/mol, besonders bevorzugt von 250 bis 12000 g/mol aufweisen, eingesetzt. Ein breiter Überblick über geeignete polymere Polyole B) findet sich beispielsweise in N. Adam et al.: "Polyurethanes", Ullmann's Encyclopedia of Industrial Chemistry, Electronic Release, 7th ed., chap. 3.2 - 3.4, Wiley-VCH, Weinheim 2005.

Geeignete Polyetherpolyole B) sind beispielsweise solche der in der DE 26 22 951 B, Spalte 6, Zeile 65 bis Spalte 7, Zeile 26, der EP-A 0 978 523 Seite 4, Zeile 45 bis Seite 5, Zeile 14 oder der WO 2011/069966, Seite 4, Zeile 20 bis Seite 5, Zeile 23 genannten Art, sofern sie den oben gemachten Angaben hinsichtlich Funktionalität und Molekulargewicht entsprechen. Besonders bevorzugte Polyetherpolyole B) sind Anlagerungsprodukte von Ethylenoxid und/oder Propylenoxid an 1,2-Propandiol, 1,3-Propandiol, Glycerin, Trimethylolpropan, Ethylendiamin und/oder Pentaerythrit oder die beispielsweise gemäß Angew. Chem. 72, 927 (1960) durch Polymerisation von Tetrahydrofuran erhältlichen Polytetramethylenetherglykole mit zahlenmittleren Molekuargewichten von 400 g/mol bis 4000 g/mol.

Geeignete Polyesterpolyole B) sind die beispielsweise solche der in der EP-A 0 978 523, Seite 5, Zeilen 17 bis 47 oder der EP-A 0 659 792, Seite 6, Zeilen 32 bis 45 genannten Art, sofern sie den oben gemachten Angaben hinsichtlich Funktionalität und Molekulargewicht entsprechen. Besonders bevorzugte Polyesterpoylole sind Kondensationsprodukte mehrwertiger Alkohole, wie z. B. 1,2-Ethandiol, 1,2-Propandiol, Diethylenglykol, 1,4-Butandiol, 1,6-Hexandiol, Neopentylglykol, 1,4-Cyclohexandimethanol, 1,4-Cyclohexandiol, Perhydrobisphenol, 1,1,1-Trimethylolpropan, 1,2,3-Propantriol, Pentaerythrit und/oder Sorbitol, mit unterschüssigen Mengen an mehrwertigen Carbonsäuren bzw. Carbonsäureanhydriden, wie z. B. Bernsteinsäure, Adipinsäure, Sebazinsäure, Dodecandisäure, Glutarsäureanhydrid, Maleinsäureanhydrid, Phthalsäureanhydrid, Isophthalsäure, Terephthalsäure, Trimellitsäure Hexahydrophthalsäureanhydrid und/oder Tetrahydrophthalsäureanhydrid, oder solche, wie sie in an sich bekannter Weise aus Lactonen, wie z. B. ε-Caprolacton, und einfachen mehrwertigen Alkoholen, wie z. B. den oben beispielhaft genannten, als Startermolekülen unter Ringöffnung erhältlich sind.

Geeignete Polycarbonatpolyole B) sind insbesondere die an sich bekannten Umsetzungsprodukte zweiwertiger Alkohole, beispielsweise solcher, wie sie oben in der Liste der mehrwertigen Alkohole beispielhaft genannt sind, mit Diarylcarbonaten, wie z. B. Diphenylcarbonat, Dimethylcarbonat oder Phosgen. Geeignete Polycarbonatpolyole B) sind auch solche, die neben Carbonatstrukturen zusätzlich Estergruppen enthalten. Hierbei handelt es sich insbesondere um die an sich bekannten Polyestercarbonatdiole, wie sie beispielsweise gemäß der Lehre der DE-AS 1 770 245 durch Umsetzung zweiwertiger Alkohole mit Lactonen, wie insbesondere ε-Caprolacton, und anschließende Reaktion der dabei entstehenden Polyesterdiole mit Diphenyl- oder Dimethylcarbonat erhalten werden können. Ebenfalls geeignete Polycarbonatpolyole B) sind solche, die neben Carbonatstrukturen zusätzlich Ethergruppen enthalten. Hierbei handelt es sich insbesondere um die an sich bekannten Polyethercarbonatpolyole, wie sie beispielsweise nach dem Verfahren der EP-A 2046861 durch katalytische Umsetzung von Alkylenoxiden (Epoxiden) und Kohlendioxid in Anwesenheit von H-funktionellen Startersubstanzen erhältlich sind.

Geeignete Polyacrylatpolyole B) sind beispielsweise solche der in WO 2011/124710 Seite 10, Zeile 32 bis Seite 13, Zeile 18 genannten Art, sofern sie den oben gemachten Angaben hinsichtlich Funktionalität und Molekulargewicht entsprechen. Besonders bevorzugte Polyacrylatpolyole B) sind Polymerisate bzw. Copolymerisate von Hydroxyalkylestern der Acrylsäure oder Methacrylsäure, wie z. B. Hydroxyethyl(meth)acrylat, Hydroxypropyl(meth)acrylat oder Hydroxybutyl(meth)acrylat, gegebenenfalls gemeinsam mit Acrylsäurealkylestern und/oder Methacrylsäurealkylestern, wie z. B. Methyl(meth)acrylat, Ethyl(meth)acrylat, n-Butyl(meth)acrylat, iso-Butyl(meth)acrylat, 2-Ethylhexyl(meth)acrylat, Cyclohexyl(meth)acrylat, Isobornyl(meth)acrylat, Lauryl(meth)acrylat, Styrol oder anderen copolymerisierbaren olefinisch ungesättigten Monomeren, wie z. B. Acrylsäure, Methacrylsäure oder Maleinsäuredimethylester.

Geeignete Polyole B) sind beispielsweise auch die bekannten, durch Umsetzung einfacher Glykole, wie z. B. Diethylenglykol, Triethylenglykol, 4,4'-Dioxethoxy-diphenyl-dimethylmethan (Addukt von 2 mol Ethylenoxid an Bisphenol A) oder Hexandiol, mit Formaldehyd erhältlichen Polyacetalpolyole oder auch durch Polykondensation cyclischer Acetale, wie z. B. Trioxan, hergestellte Polyacetale.

Weitere geeignete Polyole B) sind beispielsweise auch die in EP-A 0 689 556 und EP-A 0 937 110 beschriebenen, z. B. durch Umsetzung epoxidierter Fettsäureester mit aliphatischen oder aromatischen Polyolen unter Epoxidringöffung erhältlichen speziellen Polyole.

Hydroxylgruppen enthaltende Polybutadiene können ebenfalls als Polyole B) dienen.

In einer bevorzugten Ausführungsform der Erfindung werden als Komponente B) Polyether-, Polyester-, Polycarbonat- und/oder Polyacrylatpolyole eingesetzt.

Die Polyole B) kommen beim erfindungsgemäßen Verfahren einzeln oder in Form beliebiger Mischungen untereinander zum Einsatz. Sie können sowohl in lösemittelfreier Form als auch in üblichen Lösemitteln gelöst vorliegen.

Geeignete Lösemittel sind insbesondere solche, die sich gegenüber der Isocyanatgruppen der Komponente A) inert verhalten, beispielsweise die bekannten üblichen aprotischen Lacklösemittel, wie z. B. Ethylacetat, Isopropylacetat, Butylacetat, Isobutylacetat, Amylacetat, 2-Ethylhexylacetat, Ethylenglykolmonomethyletheracetat, Ethylenglykolmonoethyletheracetat, Ethylenglykolmonobutyletheracetat, 1-Methoxypropyl-2-acetat, 3-Methoxy-n-butylacetat, Aceton, Diethylketon, 2-Butanon, 4-Methyl-2-pentanon, Diisobutylketon Cyclohexanon, Cyclohexan, Toluol, Xylol, Chlorbenzol, Dichlorbenzol, Testbenzin, höher substituierte Aromaten, wie sie beispielsweise unter den Bezeichnungen Solventnaphtha, Solvesso®, Isopar®, Nappar® (Deutsche EXXON CHEMICAL GmbH, Köln, DE) und Shellsol® (Deutsche Shell Chemie GmbH, Eschborn, DE) im Handel sind, aber auch Lösemittel wie Propylenglykoldiacetat, Diethylenglykoldimethylether, Dipropylenglykoldimethylether, Diethylenglykolethyl- und -butyletheracetat, Ethylethoxypropionat, Propylencarbonat, N-Methylpyrrolidon und N-Methylcaprolactam, Dioxan, Tetrahydrofuran, 1,1,2,2-Tetramethoxyethan oder beliebige Gemische solcher Lösemittel.

Zur Durchführung des erfindungsgemäßen Verfahrens wird bevorzugt wenigstens eine Zusammensetzung von Isocyanatgruppen und cyclische Carbonatstukturen enthaltenden Verbindungen A) mit mindestens einem Polyol B) in beliebiger Reihenfolge bei Temperaturen von 20 bis 200°C, vorzugsweise von 40 bis 160°C, besonders bevorzugt von 60 bis 120°C, gegebenenfalls in Gegenwart mindestens eines der vorstehend genannten aprotischen Lösemittel zu cyclische Carbonatstrukturen enthaltenden Polyurethanen umgesetzt. Dabei wird ein Äquivalenzverhältnis von Isocyanatgruppen zu Hydroxylgruppen von 0,3 : 1 bis 1,2 : 1, vorzugsweise von 0,4 : 1 bis 1,1 : 1, besonders bevorzugt von 0,5 : 1 bis 1,05 : 1 eingehalten.

Das erfindungsgemäße Verfahren kann unkatalysiert durchgeführt werden. Zur Beschleunigung der Urethanisierungsreaktion können gegebenenfalls aber auch in der Isocyanatchemie übliche Katalysatoren mitverwendet werden. Geeignete Urethanisierungskatalysatoren sind beispielsweise die bereits oben als zur Herstellung der Zusammensetzung A) geeignet beschriebenen Verbindungen. Diese Katalysatoren können auch beim erfindungsgemäßen Verfahren einzeln oder in Form beliebiger Mischungen untereinander eingesetzt werden und kommen dabei, falls überhaupt, in Mengen von 0,001 bis 1,0 Gew.-%, bevorzugt 0,01 bis 0,5 Gew.-%, berechnet als Gesamtmenge an eingesetzten Katalysatoren bezogen auf die Gesamtmenge der Reaktionspartner A) und B), zum Einsatz.

In einer bevorzugten Ausführungsform des erfindungsgemäßen Verfahrens wird die Zusammensetzung A) gegebenenfalls unter Inertgas, wie beispielsweise Stickstoff, und gegebenenfalls in Gegenwart eines geeigneten Lösungsmittels der genannten Art bei einer Temperatur zwischen 20 und 100°C vorgelegt. Anschließend wird ein Polyol B) oder eine Mischung von Polyolen B) gegebenenfalls gemeinsam mit weiterem Lösungsmittel in beliebiger Reihenfolge nacheinander oder im Gemisch im oben angegebenen Äquivalentverhältnis zugegeben und die Reaktionstemperatur für die Urethanisierung gegebenenfalls durch eine geeignete Maßnahme (Heizen oder Kühlen) auf eine Temperatur von 40 bis 160°C eingestellt. Bei Mitverwendung von Katalysatoren können diese der Zusammensetzung A) und/oder der Polyolkomponente B) bereits vor Beginn der eigentlichen Umsetzung zugesetzt werden. Es ist aber auch möglich, die Katalysatoren dem Reaktionsgemisch zu einem beliebigen Zeitpunkt während der Urethanisierungsreaktion zuzusetzen.

Der Verlauf der Umsetzung kann beim erfindungsgemäßen Verfahren durch z. B. titrimetrische Bestimmung des NCO-Gehaltes oder IR-spektroskopisch verfolgt werden. Im Anschluss an die Urethanisierungsreaktion, d. h. nach vollständigem Umsatz von Isocyanat- und Hydroxylgruppen, erhält man als Produkte des erfindungsgemäßen Verfahrens die erfindungsgemäßen cyclische Carbonatstrukturen enthaltenden Polyurethane.

Ein weiterer Gegenstand der Erfindung sind cyclische Carbonatstrukturen enthaltende Polyurethane, erhältlich nach dem erfindungsgemäßen Verfahren.

Die erfindungsgemäßen cyclische Carbonatstrukturen enthaltenden Polyurethane stellen wertvolle Bindemittel zur Herstellung von Lack-, Dichtstoff- oder Klebstoffrohstoffen dar.

Weitere Gegenstände der Erfindung sind die Verwendung der erfindungsgemäßen cyclische Carbonatstrukturen enthaltenden Polyurethane als Ausgangskomponente bei der Herstellung von vernetzbaren Bindemitteln oder von Lack-, Dichtstoff- oder Klebstoffrohstoffen, sowie die die erfindungsgemäßen cyclische Carbonatstrukturen enthaltenden Polyurethane enthaltenden vernetzbaren Bindemittel und Lack-, Dichtstoff- oder Klebstoffrohstoffe.

Die erfindungsgemäßen cyclische Carbonatstrukturen enthaltenden Polyurethane können bevorzugt als Bindemittel zur Herstellung von Lack-, Dichtstoff- oder Klebstoffrohstoffen entweder lösemittelfrei eingesetzt werden, lassen sich bei Bedarf aber auch mit üblichen Lösemitteln, beispielsweise den obengenannten, beim erfindungsgemäßen Verfahren gegebenenfalls mitzuverwendenden inerten Lacklösemitteln, trübungsfrei verdünnen.

Während sich die erfindungsgemäßen Verfahrensprodukte auf Basis einfacher Alkohole und/oder verzweigter Polyole, insbesondere von Polyester- und/oder Polyacrylatpolyolen, vorzugsweise für klassische Lackanwendungen eignen, kommen solche auf Basis hochmolekularer linearer Polyetherdiole insbesondere für Anwendungen im Dichtstoff- oder Klebstoffbereich zum Einsatz.

Erfindungsgemäße Verfahrensprodukte, bei deren Herstellung die Polyolkomponente B) mit einem Unterschuss an Isocyanatgruppen insbesondere in einem Äquivalentverhältniss von Isocyanatgruppen zu Hydroxylgruppen von 0,3 : 1 bis 0,7 : 1, vorzugsweise von 0,4 : 1 bis 0,6 : 1, besonders bevorzugt von 0,45 : 1 bis 0,55 : 1, umgesetzt wurde, können als alleinige Bindemittel dienen, da sie sowohl cyclische Carbonatstrukturen als auch nicht umgesetzte Hydroxylgruppen und damit gleichzeitig zwei miteinander reaktionsfähige Gruppen aufweisen.

Erfindungsgemäßen Verfahrensprodukten, bei deren Herstellung die Polyolkomponente B) mit einem geringeren Unterschuss oder sogar einem leichten Überschuss an Isocyanatgruppen, insbesondere in einem Äquivalentverhältniss von Isocyanatgruppen zu Hydroxylgruppen von 0,7 : 1 bis 1,2 : 1, vorzugsweise von 0,8 : 1 bis 1,1 : 1, besonders bevorzugt von 0,9 : 1 bis 1,05 : 1, umgesetzt wurde, werden zur Vernetzung in der Regel geeignete hydroxy- und/oder aminofunktionelle Reaktionspartner zugesetzt, mit denen sie unter Ringöffnung der Cyclocarbonatstrukturen reagieren können.

Geeignete hydroxyfunktionelle Reaktionspartner zur Aushärtung der erfindungsgemäßen cyclische Carbonatstrukturen enthaltenden Polyurethane sind grundsätzliche alle bereits oben als geeignete Polyolkomponenten B) zur Herstellung dieser Polyurethane genannten Verbindungen.

Geeignete aminofunktionelle Reaktionspartner sind beispielsweise beliebige aliphatische und cycloaliphatische Amine mit mindestens zwei primär und/oder sekundär gebundenen Aminogruppen, wie z. B. 1,2-Diaminoethan, 1,2-Diaminopropan, 1,3-Diaminopropan, 1,4-Diaminobutan, 1,2-Diamino-2-methylpropan, 1,5-Diaminopentan, 1,3-Diamino-2,2-dimethylpropan, 1,6-Diaminohexan, 1,5-Diamino-2-methylpentan, 1,6-Diamino-2,2,4-trimethylhexan, 1,6-Diamino-2,4,4-trimethylhexan, 1,7-Diaminoheptan, 1,8-Diaminooctan, 2,5-Diamino-2,5-dimethylhexan, 1,9-Diaminononan, 2-Methyl-1,8-diaminooctan, 1,10-Diaminodecan, 1,11-Diaminoundecan, 1,12-Diaminododecan, 1,2-Diaminocyclopentan, 1,2-Diaminocyclohexan, 1-Amino-3,3,5-trimethyl-5-aminomethylcyclohexan (Isophorondiamin, IPDA), 3(4)-Aminomethyl-1-methylcyclohexylamin, 1,3-Diamino-2- und/oder -4-methylcyclohexan, Isopropyl-2,4- und/oder 2,6-diaminocyclohexan, 1,3-Bis(aminomethyl)-cyclohexan, 1,8-p-Diaminomenthan, Bis(4-aminocyclohexyl)-methan, Bis(4-amino-3-methylcyclohexyl)-methan, Bis(4-amino-3,5-di-methylcyclohexyl)-methan, Bis(4-amino-2,3,5-trimethylcyclohexyl)-methan, 1,1-Bis(4-aminocyclohexyl)-propan, 2,2-Bis(4-aminocyclohexyl)-propan, 1,1-Bis(4-aminocyclohexyl)-ethan, 1,1-Bis(4-aminocyclohexyl)-butan, 2,2-Bis(4-aminocyclohexyl)-butan, 1,1-Bis(4-amino-3-methylcyclohexyl)-ethan, 2,2-Bis(4-amino-3-methylcyclohexyl)-propan, 1,1-Bis(4-amino-3,5-dimethylcyclohexyl)-ethan, 2,2-Bis(4-amino-3,5-dimethylcyclohexyl)-propan, 2,2-Bis(4-amino-3,5-dimethylcyclohexyl)-butan, 2,4-Diaminodicyclohexylmethan, 4-Aminocyclohexyl-4-amino-3-methylcyclohexyl-methan, 4-Amino-3,5-dimethylcyclohexyl-4-amino-3-methylcyclohexylmethan und 2-(4-Amino-cyclohexyl)-2-(4-amino-3-methylcyclohexyl)-methan, m-Xylylendiamin, Methyliminobispropylamin, Iminobispropylamin, Bis(6-aminohexyl)-amin, N,N-Bis(3-aminopropyl)-ethylendiamin, 4-Aminomethyl-1,8-octandiamin, Bis(aminopropyl)piperazin, Aminoethylpiperazin, Diethylentriamin, Dipropylentriamin, Triethylentetramin, Tetraethylenpentamin, Pentaethylenhexamin, Heptaethylenoctamin, oder beliebige Gemische solcher Polyamine.

Geeignete aminofunktionelle Reaktionspartner für die erfindungsgemäßen cyclische Carbonatstrukturen enthaltenden Polyurethane sind auch aminofunktionelle Polyalkylenglykole, wie z. B. 1,2-Bis(aminoethoxy)-ethan, 1,11-Diamino-3,6,9-trioxaundecan, 1,13-Diamino-4,7,10-trioxatride- can und insbesondere die unter dem Handelsnamen Jeffamine^{®} von Firma Huntsman Corp. kommerziell vertriebenen aminfunktionalisierten Polyalkylenglykole mit zahlenmittleren Molekulargewichten Mₙ bis zu 5000 g/mol, bevorzugt bis zu 2000 g/mol, besonders bevorzugt bis zu 1000 g/mol.

Gegebenenfalls können auch sterisch gehinderte aliphatische Diamine mit zwei sekundär gebundenen Aminogruppen als Reaktionspartner für die erfindungsgemäßen cyclische Carbonatstrukturen enthaltenden Polyurethane eingesetzt werden, wie z. B. die aus EP-A 0 403 921 bekannten Umsetzungsprodukte aliphatischer und/oder cycloaliphatischer Diamine mit Maleinsäure- oder Fumarsäureestern, das gemäß der Lehre der EP-A 1 767 559 erhältliche Bisaddukt von Acrylnitril an Isophorondiamin oder die beispielsweise in der DE-A 19 701 835 beschriebenen Hydrierungsprodukte aus aliphatischen und/oder cycloaliphatischen Diaminen und Ketonen, wie z. B. Diisopropylketon, zugänglicher Schiffscher Basen.

Weitere für die erfindungsgemäßen Verfahrensprodukte als Reaktionspartner geeigneten Polyamine sind darüberhinaus auch die als Vernetzerkomponenten für Epoxyharze bekannten Polyamidoamine, Polyimine und/oder Polyvinylamine.

Als Reaktionspartner für die erfindungsgemäßen Verfahrensprodukte sind schließlich auch Aminoalkohole, wie z. B. 2-Aminoethanol, die isomeren Aminopropanole und -butanole, 3-Amino-1,2-propandiol und 1,3- Diamino-2-propanol, geeignet.

Bei der Formulierung von Lacken, Dichtstoffen oder Klebstoffen aus den erfindungsgemäßen cyclische Carbonatstrukturen enthaltenden Polyurethanen werden die genannten hydroxy- und/oder aminofunktionellen Reaktionspartner entweder als Einzelverbindungen oder in Form beliebiger Gemische untereinander in der Regel in solchen Mengen zugesetzt, dass auf jede Cyclocarbonatstruktur von 0,7 bis 1,5, vorzugsweise von 0,8 bis 1,2, besonders bevorzugt 0,9 bis 1,1 Hydroxyl- und/oder Aminogruppen entfallen.

Die Aushärtung der aus den erfindungsgemäßen cyclische Carbonatstrukturen enthaltenden Polyurethanen hergestellten Lacke, Dichtstoffe oder Klebstoffe kann über einen weiten Temperaturbereich, beispielsweise von -20°C bis 250°C, vorzugsweise von 0°C bis 200°C, unkatalysiert oder in Gegenwart geeigneter Katalysatoren erfolgen. Während die Reaktion mit aminischen Reaktionspartnern in der Regel bereits bei niedrigen Temperaturen und ohne Katalysatorzusatz ausreichend schnell abläuft, benötigt die Aushärtung mit alkoholischen Reaktionspartnern für eine vollständige Vernetzung höhere Temperaturen und häufig die Mitverwendung geeigneter Katalysatoren. Geeignete Katalysatoren für die Aushärtung der erfindungsgemäßen cyclische Carbonatstrukturen enthaltenden Polyurethane mit hydroxy- und/oder aminofunktionellen Reaktionspartnern sind beispielsweise die bereits oben als zur Herstellung der Isocyanatgruppen und cyclische Carbonatstrukturen enthaltenden Verbindungen A) geeignet beschriebenen Verbindungen. Weitere ebenfalls als Katalysatoren geeignete Verbindungen sind daneben auch die in der EP-A 0 911 352 als Katalysatoren genannten organometallischen Verbindungen und Metallcarboxylate, sowie die in der EP-A 0 983 231 beschriebenen Basen, deren konjugierte Säuren einen pKa von 13 oder mehr aufweisen, insbesondere die dort genannten Hydroxide, Alkoholate und/oder N,N-Bis(trimethylsilyl)amidate.

Diese Katalysatoren können einzeln oder in Form beliebiger Mischungen untereinander eingesetzt werden und kommen dabei, falls überhaupt, in Mengen von 0,001 bis 3,0 Gew.-%, bevorzugt 0,01 bis 1,0 Gew.-%, berechnet als Gesamtmenge an eingesetzten Katalysatoren bezogen auf die Gesamtmenge an erfindungsgemäßen cyclische Carbonatstrukturen enthaltenden Polyurethane und gegebenenfalls mitverwendeten alokoholischen und/oder aminischen Reaktionspartnern, zum Einsatz.

Den erfindungsgemäßen cyclische Carbonatstrukturen enthaltenden Polyurethane können bei der Formulierung von Lacken, Dichtstoffen oder Klebstoffen auch beliebige weitere Hilfs- und Zusatzmittel, wie z. B. die üblichen UV-Stabilisatoren, Antioxidantien, Wasserfänger, Slipadditive, Entschäumer, Verlaufsmittel, Rheologieadditive, Flammschutzmittel, Füllstoffe und/oder Pigmente, zugesetzt werden.

Die Applikation der unter Verwendung der erfindungsgemäßen cyclische Carbonatstrukturen enthaltenden Polyurethane formulierten Beschichtungen, Dichtstoffe oder Klebstoffe kann nach an sich bekannten Methoden erfolgen, beispielsweise durch Spritzen, Streichen, Tauchen, Fluten oder mit Hilfe von Walzen oder Rakeln in einer oder mehrerer Schichten. Als Untergründe kommen dabei beliebige Substrate in Betracht, wie z. B. Metall, Holz, Glas, Stein, keramische Materialien, Beton, harte und flexible Kunststoffe, Textilien, Leder und Papier, die vor der Beschichtung gegebenenfalls auch mit üblichen Grundierungen versehen werden können.

### Beispiele

Die Erfindung wird im Folgenden an Hand von Beispielen näher erläutert.

Alle Prozentangaben beziehen sich, soweit nichts Anderslautendes vermerkt, auf das Gewicht.

Die Bestimmung der NCO-Gehalte erfolgte titrimetrisch nach DIN EN ISO 11909.

OH-Zahlen wurden titrimetrisch nach DIN 53240-2: 2007-11, Säure-Zahlen nach DIN 3682 bestimmt. Die angegebenen OH-Gehalte wurde aus den analytisch ermittelten OH-Zahlen errechnet.

Die Rest-Monomeren Gehalte wurden nach DIN EN ISO 10283 gaschromatographisch mit internem Standard gemessen.

Die Anteile an Bisaddukt (aus zwei Molekülen hydroxyfunktionellem cyclischen Carbonat und einem Molekül Diisocyanat) wurden durch Gelpermationschromatographie in Anlehnung an DIN 55672-1 (Gelpermeationschromatographie (GPC) - Teil 1: Tetrahydrofuran (THF) als Elutionsmittel) ermittelt, mit der Änderung, dass mit einer Flußrate von 0,6 ml/min statt 1,0 ml/min gearbeitet wurde. Die den Chromatogrammen entnommenen Anteile an Bisaddukt in Flächen-% wurden näherungsweise jeweils Anteilen in Gew.-% gleichgesetzt und als solche, bezogen auf die Gesamtmenge an Mono- und Bisaddukt, angegeben.

Sämtliche Viskositätsmessungen erfolgten mit einem Physica MCR 51 Rheometer der Fa. Anton Paar Germany GmbH (DE) nach DIN EN ISO 3219.

Die angegebenen Schmelzbereiche wurden mit Hilfe einer Kofler-Heizbank der Fa. Wagner & Munz GmbH (DE) ermittelt.

### Ausgangsverbindungen

### Herstellung der Zusammensetzungen A)

### Zusammensetzung A1)

1680 g (10 mol) Hexamethylendiisocyanat (HDI) wurden bei einer Temperatur von 100°C unter trockenem Stickstoff vorgelegt, innerhalb von 30 Minuten mit 118 g (1 mol) Glycerincarbonat versetzt und weitere 5 Stunden gerührt, bis ein NCO-Gehalt von 44,4 %, entsprechend einer vollständigen Urethanisierung, erreicht war. Anschließend wurde das nicht umgesetzte monomere HDI bei einer Temperatur von 140°C und einem Druck von 0,1 mbar im Dünnschichtverdampfer abgetrennt. Man erhielt ein praktisch farbloses, klares isocyanatfunktionelles cyclisches Carbonat, das bei Raumtemperatur innerhalb einer Woche langsam durchkristallisierte.

Das Produkt wies die folgenden Kenndaten auf:

| | |
|---|---|
| NCO-Gehalt: | 14,1 % |
| monomeres HDI: | 0,19 % |
| Schmelzbereich: | 28 - 30°C |
| Anteil Bisaddukt: | 3,8 % |

### Zusammensetzung A2)

Nach dem für Zusammensetzung A1) beschriebenen Verfahren wurden 1776 g (8 mol) Isophorondiisocyanat (IPDI) mit 118 g (1 mol) Glycerincarbonat umgesetzt. Nach Erreichen eines NCO-Gehalts von 33,3 %, entsprechend einer vollständigen Urethanisierung, wurde das nicht umgesetzte monomere IPDI bei einer Temperatur von 160°C und einem Druck von 0,2 mbar durch Dünnschichtdestillation entfernt und man erhielt ein isocyanatfunktionelles cyclisches Carbonat in Form eines schwach gelb gefärbten, klaren Festharzes.

| | |
|---|---|
| NCO-Gehalt: | 11,8 % |
| monomeres IPDI: | 0,21 % |
| Anteil Bisaddukt: | 4,5 % |

### Zusammensetzung A3)

Nach dem für Zusammensetzung A1) beschriebenen Verfahren wurden 2096 g (8 mol) 4,4'-Diisocyanatodicyclohexylmethan (H₁₂-MDI) mit 118 g (1 mol) Glycerincarbonat umgesetzt. Nach Erreichen eines NCO-Gehalts von 28,5 %, entsprechend einer vollständigen Urethanisierung, wurde das nicht umgesetzte monomere H₁₂-MDI bei einer Temperatur von 170°C und einem Druck von 0,2 mbar durch Dünnschichtdestillation entfernt und man erhielt ein isocyanatfunktionelles cyclisches Carbonat in Form eines schwach gelb gefärbten, klaren Festharzes.

| | |
|---|---|
| NCO-Gehalt: | 10,3 % |
| monomeres H12-MDI: | 0,28 % |
| Anteil Bisaddukt: | 4,6 % |

### Zusammensetzung A4)

Nach dem für Zusammensetzung A1) beschriebenen Verfahren wurden 1344 g (8 mol) HDI mit 160 g (1 mol) TMP-Carbonat umgesetzt. Nach Erreichen eines NCO-Gehalts von 41,9 %, entsprechend einer vollständigen Urethanisierung, wurde das nicht umgesetzte monomere HDI bei einer Temperatur von 160°C und einem Druck von 0,2 mbar durch Dünnschichtdestillation entfernt und man erhielt ein fast farbloses, klares isocyanatfunktionelles cyclisches Carbonat mit folgenden Kenndaten:

| | |
|---|---|
| NCO-Gehalt: | 12,1 % |
| monomeres HDI: | 0,17 % |
| Viskosität (23°C): | 77000 mPas |
| Anteil Bisaddukt: | 5,1 % |

### Zusammensetzung A5)

Nach dem für Zusammensetzung A1) beschriebenen Verfahren wurden 672 g (4 mol) HDI mit 118 g (1 mol) Glycerincarbonat umgesetzt. Nach Erreichen eines NCO-Gehalts von 37,2 %, entsprechend einer vollständigen Urethanisierung, wurde das nicht umgesetzte monomere HDI wie in Beispiel 1 beschrieben durch Dünnschichtdestillation entfernt und man erhielt ein praktisch farbloses, klares isocyanatfunktionelles cyclisches Carbonat, das bei Raumtemperatur innerhalb einer Woche langsam durchkristallisierte.

Das Produkt wies die folgenden Kenndaten auf:

| | |
|---|---|
| NCO-Gehalt: | 13,4 % |
| monomeres HDI: | 0,11 % |
| Schmelzbereich: | 27 - 30°C |
| Anteil Bisaddukt: | 9,1 % |

### Zusammensetzung A6) (Vergleich)

Nach dem für Zusammensetzung A1) beschriebenen Verfahren wurden 504 g (3 mol) HDI mit 118 g (1 mol) Glycerincarbonat umgesetzt. Nach Erreichen eines NCO-Gehalts von 33,8 %, entsprechend einer vollständigen Urethanisierung, wurde das nicht umgesetzte monomere HDI wie in Beispiel 1 beschrieben durch Dünnschichtdestillation entfernt und man erhielt ein praktisch farbloses, klares isocyanatfunktionelles cyclisches Carbonat, das nach Abkühlung auf Raumtemperatur teilweise kristallisierte.

Das Produkt wies die folgenden Kenndaten auf:

| | |
|---|---|
| NCO-Gehalt: | 127 % |
| monomeres HDI: | 0,19 % |
| Anteil Bisaddukt: | 13,5 % |

### Polyole B

### Polyol B1)

70 %ig in Butylacetat gelöstes Polyacrylatpolyol, hergestellt aus 33,0 % Hydroxyethylmethacrylat, 24,3 % n-Butylacrylat, 38,8 % Styrol, 0,9 % Acrylsäure und 3,0 % Di-tert.-butylperoxid.

| | |
|---|---|
| OH-Zahl (OH-Gehalt) | 98 mg KOH/g (3,0 %) |
| Äquivalentgewicht: | 572 g/val OH |
| Säurezahl: | 7,5 mg KOH/g |
| Viskosität (23°C): | 3.500 mPas |

### Polyol B2)

Lösemittelfreies Polyesterpolyol, hergestellt aus 11,9 % Adipinsäure, 33,7 % Isophthalsäure, 10,7 % Trimethylolpropan, 37,7 % 1,6-Hexandiol und 6,0 % Phthalsäureanhydrid.

| | |
|---|---|
| OH-Zahl (OH-Gehalt) | 143 mg KOH/g (4,3 %) |
| Äquivalentgewicht: | 392 g/val OH |
| Säurezahl: | 1 mg KOH/g |
| Viskosität (23°C): | 2.500 mPas (als 80 %ige Lösung in Butylacetat) |

### Polyol B3)

75 %ig in Solventnaphtha 100 gelöstes Polyesterpolyol, hergestellt aus 19,2 % Adipinsäure, 22,3 % Maleinsäureanhydrid, 4,6 % Trimethylolpropan, 1,7 % 1,2-Propandiol und 40,2 % Neopentylglykol.

| | |
|---|---|
| OH-Zahl (OH-Gehalt) | 68 mg KOH/g (2,0 %) |
| Äquivalentgewicht: | 825 g/val OH |
| Säurezahl: | 3 mg KOH/g |
| Viskosität (23°C): | 3.700 mPas |

### Beispiel 1 (erfindungsgemäß und Vergleich)

297,9 g (1,0 val) der Zusammensetzung A1) wurden mit 524,3 g Butylacetat verdünnt, bei einer Temperatur von 80°C unter trockenem Stickstoff mit 565,6 g (1,0 val) des Polyols B1) versetzt und anschließend 8 Stunden bei 90°C gerührt, bis IR-spektroskopisch kein Isocyanat mehr nachweisbar war. Nach Abkühlen auf Raumtemperatur lag ein erfindungsgemäßes cyclische Carbonatstrukturen enthaltendes Polyurethan in Form einer farblosen Lösung vor.

| | |
|---|---|
| Festkörpergehalt: | 50 % |
| Viskosität (23°C): | 1.370 mPas |
| Äquivalentgewicht: | 1387,8 g/val cyclisches Carbonat |

Zum Vergleich wurden 1680 g (10 mol) Hexamethylendiisocyanat (HDI) bei einer Temperatur von 80°C unter trockenem Stickstoff innerhalb von 20 Minuten mit 283 g (0,5 val) des Polyols B1) versetzt. Die Temperatur wurde anschließend auf 100°C erhöht und das Reaktionsgemisch weiter gerührt, bis nach 2 Stunden ein NCO-Gehalt von 41,7 %, entsprechend einer vollständigen Urethanisierung, erreicht war. Der Versuch, das nicht umgesetzte überschüssige monomere HDI gemeinsam mit dem aus Polyol B1) stammenden Butylacetat durch Dünnschichtdestillation abzutrennen, scheiterte. Bei einer Temperatur von 140°C und einem Druck von 0,1 mbar bildete sich auf der Verdampferfläche ein festes, nicht fließfähiges Harz.

Der Vergleich zeigt, dass sich aus Polyol B1) und HDI kein monomerarmes isocyanatfunktionelles Prepolymer herstellen läßt, das sich mit Glycerincarbonat zu einem cyclische Carbonatstrukturen enthaltenden Polyurethan ähnlich dem erfindungsgemäß erhaltenen umsetzen ließe.

### Beispiel 2 (erfindungsgemäß)

282 g (0,9 mol) der Zusammensetzung A5) wurden unter trockenem Stickstoff in 215 g 1,1,2,2-Tetramethoxyethan als Lösungsmittel bei einer Temperatur von 80°C vorgelegt, innerhalb von 20 min portionsweise mit 40 g (0,3 mol) Trimethylolpropan (TMP) versetzt und weitere 4 Stunden bis zum vollständigen Verschwinden der Isocyanatbande im IR-Spektrum gerührt. Nach Abkühlen auf Raumtemperatur lag ein erfindungsgemäßes cyclische Carbonatstrukturen enthaltendes Polyurethan in Form einer farblosen Lösung vor.

| | |
|---|---|
| Festkörpergehalt: | 60 % |
| Viskosität (23°C): | 5.220 mPas |
| Äquivalentgewicht: | 596,7 g/val cyclisches Carbonat |

### Beispiel 3 (Vergleich)

298 g (0,9 mol) der nicht erfindungsgemäßen Zusammensetzung A6) wurden unter trockenem Stickstoff in 225 g 1,1,2,2-Tetramethoxyethan als Lösungsmittel bei einer Temperatur von 80°C vorgelegt, innerhalb von 20 min portionsweise mit 40 g (0,3 mol) Trimethylolpropan (TMP) versetzt und weitere 4 Stunden bis zum vollständigen Verschwinden der Isocyanatbande im IR-Spektrum gerührt. Die vorliegende 60 %ige Lösung eines endständig Cyclocarbonatgruppen tragenden Polyurethans trübte unmittelbar nach Abkühlen auf Raumtemperatur stark ein und bildete innerhalb eines Tages einen deutlichen Bodensatz.

Der Vergleich mit dem nach Beispiel 2) als klare Lösung erhaltenen Umsetzungsprodukt der erfindungsgemäßen Zusammensetzung A5) mit TMP zeigt, dass die Zusasmmensetzung A6), die unter Verwendung eines geringeren als dem erfindungsgemäßen Mindestüberschuss an Isocyanatgruppen hergestellt wurde, aufgrund eines zu hohen Anteils an Bisaddukt nicht kristallisationsstabil und somit zur Herstellung Cyclocarbonatgruppen tragender Polyurethane ungeeignet ist.

### Beispiel 4 (erfindungsgemäß)

355,9 g (1,0 val) der Zusammensetzung A2) wurden mit 582,3 g Butylacetat verdünnt, bei einer Temperatur von 80°C unter trockenem Stickstoff mit 565,6 g (1,0 val) des Polyols B1) versetzt und anschließend 20 Stunden bei 100°C gerührt, bis IR-spektroskopisch kein Isocyanat mehr nachweisbar war. Nach Abkühlen auf Raumtemperatur lag ein erfindungsgemäßes cyclische Carbonatstrukturen enthaltendes Polyurethan in Form einer praktisch farblosen Lösung vor.

| | |
|---|---|
| Festkörpergehalt: | 50 % |
| Viskosität (23°C): | 1.510 mPas |
| Äquivalentgewicht: | 1503,8 g/val cyclisches Carbonat |

### Beispiel 5 (erfindungsgemäß)

355,9 g (1,0 val) der Zusammensetzung A2) wurden mit 651,8 g Butylacetat verdünnt, bei einer Temperatur von 80°C unter trockenem Stickstoff mit 493,3 g (1,0 val) einer 80 %igen Lösung des Polyols B2) in Butylacetat versetzt und anschließend 20 Stunden bei 100°C gerührt, bis IR-spektroskopisch kein Isocyanat mehr nachweisbar war. Nach Abkühlen auf Raumtemperatur lag ein erfindungsgemäßes cyclische Carbonatstrukturen enthaltendes Polyurethan in Form einer praktisch farblosen Lösung vor.

| | |
|---|---|
| Festkörpergehalt: | 50 % |
| Viskosität (23°C): | 1.490 mPas |
| Äquivalentgewicht: | 1501,0 g/val cyclisches Carbonat |

### Beispiel 6 (erfindungsgemäß)

297,9 g (1,0 val) der Zusammensetzung A1) wurden mit 722,2 g Solventnaphtha 100 verdünnt, bei einer Temperatur von 80°C unter trockenem Stickstoff mit 848,5 g (1,0 val) des Polyols B3) versetzt und anschließend 8 Stunden bei 90°C gerührt, bis IR-spektroskopisch kein Isocyanat mehr nachweisbar war. Nach Abkühlen auf Raumtemperatur lag ein erfindungsgemäßes cyclische Carbonatstrukturen enthaltendes Polyurethan in Form einer praktisch farblosen Lösung vor.

| | |
|---|---|
| Festkörpergehalt: | 50 % |
| Viskosität (23°C): | 1.860 mPas |
| Äquivalentgewicht: | 1868,6 g/val cyclisches Carbonat |

### Beispiel 7 (erfindungsgemäß)

355,9 g (1,0 val) der Zusammensetzung A2) wurden mit 780,2 g Solventnaphtha 100 verdünnt, bei einer Temperatur von 80°C unter trockenem Stickstoff mit 848,5 g (1,0 val) des Polyols B3) versetzt und anschließend 20 Stunden bei 100°C gerührt, bis IR-spektroskopisch kein Isocyanat mehr nachweisbar war. Nach Abkühlen auf Raumtemperatur lag ein erfindungsgemäßes cyclische Carbonatstrukturen enthaltendes Polyurethan in Form einer praktisch farblosen Lösung vor.

| | |
|---|---|
| Festkörpergehalt: | 50 % |
| Viskosität (23°C): | 1.980 mPas |
| Äquivalentgewicht: | 1984,6 g/val cyclisches Carbonat |

### Beispiel 8 (erfindungsgemäß)

407,8 g (1,0 val) der Zusammensetzung A3) wurden mit 634,2 g Butylacetat verdünnt, bei einer Temperatur von 80°C unter trockenem Stickstoff mit 565,6 g (1,0 val) des Polyols B1) versetzt und anschließend 20 Stunden bei 100°C gerührt, bis IR-spektroskopisch kein Isocyanat mehr nachweisbar war. Nach Abkühlen auf Raumtemperatur lag ein erfindungsgemäßes cyclische Carbonatstrukturen enthaltendes Polyurethan in Form einer leicht gelb gefärbten Lösung vor.

| | |
|---|---|
| Festkörpergehalt: | 50 % |
| Viskosität (23°C): | 2.540 mPas |
| Äquivalentgewicht: | 1607,6 g/val cyclisches Carbonat |

### Beispiel 9 (erfindungsgemäß)

347,1 g (1,0 val) der Zusammensetzung A4) wurden mit 573,5 g Butylacetat verdünnt, bei einer Temperatur von 80°C unter trockenem Stickstoff mit 565,6 g (1,0 val) des Polyols B1) versetzt und anschließend 8 Stunden bei 90°C gerührt, bis IR-spektroskopisch kein Isocyanat mehr nachweisbar war. Nach Abkühlen auf Raumtemperatur lag ein erfindungsgemäßes cyclische Carbonatstrukturen enthaltendes Polyurethan in Form einer farblosen Lösung vor.

| | |
|---|---|
| Festkörpergehalt: | 50 % |
| Viskosität (23°C): | 1.420 mPas |
| Äquivalentgewicht: | 1486,2 g/val cyclisches Carbonat |

### Beispiel 10 (erfindungsgemäß, Selbstvernetzer)

149,0 g (0,5 val) der Zusammensetzung A1) wurden mit 193,7 g Butylacetat verdünnt, bei einer Temperatur von 80°C unter trockenem Stickstoff mit 565,6 g (1,0 val) des Polyols B1) versetzt und anschließend 8 Stunden bei 90°C gerührt, bis IR-spektroskopisch kein Isocyanat mehr nachweisbar war. Nach Abkühlen auf Raumtemperatur lag ein erfindungsgemäßes cyclische Carbonatstrukturen enthaltendes und gleichzeitig hydroxyfunktionelles Polyurethan in Form einer farblosen Lösung vor.

| | |
|---|---|
| Festkörpergehalt: | 60 % |
| Viskosität (23°C): | 2.370 mPas |
| Äquivalentgewicht: | 1816,6 g/val cyclisches Carbonat |
| Äquivalentgewicht: | 1816,6 g/val OH |

### Beispiel 11 - 16 (Verwendung, erfindungsgemäß)

Aus den erfindungsgemäßen cyclische Carbonatstrukturen enthaltenden Polyurethanen 1), 4), 5) und 7) wurden unter Mitverwendung der in Tabelle 1 aufgeführten aminofunktionellen Reaktionspartner Lacke formuliert und jeweils auf einen Feststoffgehalt von 50 % eingestellt. Die Lacke wurden in einer Naßfilm-Schichtdicke von ca. 100 µm auf Glasplatten appliziert und nach 15 minütigem Ablüften bei Raumtemperatur 30 min bei 70 bzw. 120°C getrocknet. Es wurden in allen Fällen glänzende transparente Lackfilme erhalten. Die nachfolgende Tabelle 1 zeigt die Zusammensetzungen der Lackformulierungen (jeweils Gew.-Teile) sowie Eigenschaften der erhaltenen Beschichtungen.

**Tabelle 1**

| **Beispiel** | **11** | **12** | **13** | **14** | **15** | **16** |
|---|---|---|---|---|---|---|
| Polyurethan aus Beispiel 1 | 82,6 | - | - | - | - | - |
| Polyurethan aus Beispiel 4 | - | 83,6 | - | - | - | - |
| Polyurethan aus Beispiel 5 | - | - | 83,6 | 88,8 | - | - |
| Polyurethan aus Beispiel 7 | - | - | - | - | 87,2 | 91,2 |
| Jeffamin^{®} T 403 [1] | 8,7 | 8,2 | 8,2 | - | 6,4 | 4,4 |
| Tetraethylenpentamin [2] | - | - | - | 5,6 | - | - |
| Butylacetat | 8,7 | 8,2 | 8,2 | 5,6 | - | - |
| Solvent Naphta 100 | - | - | - | - | 6,4 | 4,4 |
| Vernetzungstemperatur (°C) | 120 | 120 | 120 | 70 | 70 | 120 |
| Filmoptik | i. O. | i. O. | i. O. | i. O. | i. O. | i. O. |
| Schichtdicke (µm) | 50 | 45 | 45 | 50 | 50 | 45 |
| Pendelhärte (s) nach 24/48 h [3] | 47/48 | 114/129 | 116/131 | 112/193 | 87/100 | 166/209 |
| Acetonbeständigkeit [4] | 1 | 1-2 | 2-3 | 1 | 2 | 1-2 |

| | | | | | | |
|---|---|---|---|---|---|---|
| [1] trifunktionelles Polyetheramin (Huntsman Corp., Zaventem, Belgium), Äquivalentgewicht: 146,7 g/val Amin [2] Äquivalentgewicht: 94,7 g/val Amin (bezogen auf primär gebundene Aminogruppen) [3] Pendelhärte nach König (DIN 53157) [4] Beständigkeit des ausgehärteten Lackfilmes gegen Aceton nach einer Einwirkzeit von 1 min. Bewertung: 0 - 5 (0 = Lackfilm unverändert; 1 = sichtbare Veränderung; 2 = spürbare Erweichung; 3 = starke Erweichung; 4 = bis zum Untergrund erweicht; 5 = ohne Fremdeinwirkung völlig zerstört) | | | | | | |

### Beispiele 17 (Verwendung, Selbstvernetzer)

100 Gew.-Teile des erfindungsgemäßen cyclische Carbonatstrukturen und gleichzeitig Hydroxylgruppen enthaltenden Polyurethans aus Beispiel 10) wurden mit 0,5 Gew.-Teilen Natriumlaurinat als Katalysator versetzt, durch Zusatz von 20 Gew.-Teilen Butylacetat auf einen Feststoffgehalt von 50 % eingestellt und anschließend in einer Naßfilm-Schichtdicke von ca. 100 µm auf eine Glasplatte appliziert. Nach 15 minütigem Ablüften bei Raumtemperatur wurde der Lack 30 min bei 160°C eingebrannt. Es wurde ein glänzender transparenter Lackfilm erhalten. Die Pendelhärte betrug nach 24 h 66 s, die Acetonbeständigkeit lag bei 1 - 2 (Bewertung wie in Tabelle 1, Fußnote [4] definiert).

## Patentansprüche

1. Verfahren zur Herstellung von cyclische Carbonatstrukturen enthaltenden Polyurethanen, durch Umsetzung von wenigstens
A) einer Zusammensetzung enthaltend
a) eine oder mehrere Isocyanatgruppen und cyclische Carbonatstukturen enthaltende Verbindungen der allgemeinen Formel (I) 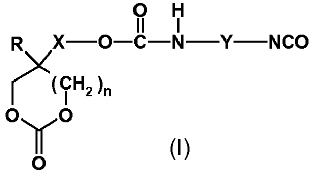 und
b) ≤ 1 Gew.-%, bezogen auf die Gesamtmasse der Zusammensetzung A), eines oder mehrerer monomerer Diisocyanate mit aliphatisch, cycloaliphatisch, araliphatisch und/oder aromatisch gebundenen Isocyanatgruppen der allgemeinen Formel (II),
**OCN-Y-NCO** (II)
wobei
R für Wasserstoff oder einen gesättigten oder ungesättigten, linearen oder verzweigten, aliphatischen Rest mit 1 bis 7 Kohlenstoffatomen steht,
X für einen linearen oder verzweigten organischen Rest mit 1 bis 36 Kohlenstoffatomen steht, der gegebenenfalls Ether-, Ester- und/oder Carbonatgruppen enthalten kann,
n für 0 oder 1 steht und
Y für einen linearen oder verzweigten, aliphatischen oder cycloaliphatischen Rest mit 4 bis 18 Kohlenstoffatomen oder einen gegebenenfalls substituierten aromatischen oder araliphatischen Rest mit 6 bis 18 Kohlenstoffatomen steht,
mit
B) einem mindestens difunktionellen Polyol mit einem zahlenmittleren Molekulargewicht Mₙ von 62 bis 22000 g/mol, bevorzugt 90 bis 12000 g/mol
unter Einhaltung eines Äquivalentverhältnisses von Isocyanatgruppen zu Hydroxylgruppen von 0,3 : 1 bis 1,2 : 1.

2. Verfahren zur Herstellung von cyclische Carbonatstrukturen enthaltenden Polyurethanen gemäß Anspruch 1, **dadurch gekennzeichnet, dass** die Zusammensetzung A) zusätzlich Verbindungen (c) der allgemeinen Formel (III) 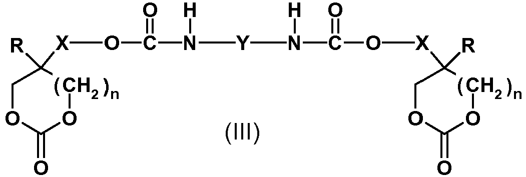 in einer Menge von 0,5 bis 12 Gew.-%, bevorzugt 0,5 bis 10 Gew.-%, bezogen auf die Gesamtmasse der Komponenten a) und c) enthält,
wobei
R für Wasserstoff oder einen gesättigten oder ungesättigten, linearen oder verzweigten, aliphatischen Rest mit 1 bis 7 Kohlenstoffatomen steht,
X für einen linearen oder verzweigten organischen Rest mit 1 bis 36 Kohlenstoffatomen steht, der gegebenenfalls Ether-, Ester- und/oder Carbonatgruppen enthalten kann,
n für 0 oder 1 steht und
Y für einen linearen oder verzweigten, aliphatischen oder cycloaliphatischen Rest mit 4 bis 18 Kohlenstoffatomen oder einen gegebenenfalls substituierten aromatischen oder araliphatischen Rest mit 6 bis 18 Kohlenstoffatomen steht.

3. Verfahren zur Herstellung von cyclische Carbonatstrukturen enthaltenden Polyurethanen gemäß Anspruch 1, **dadurch gekennzeichnet, dass** die Zusammensetzung A)
a) ≥ 88 Gew.-%, bevorzugt ≥ 90 Gew.-% an Verbindungen der allgemeinen Formel (I),
b) ≤ 1 Gew.-% eines oder mehrerer monomerer Diisocyanate mit aliphatisch, cycloaliphatisch, araliphatisch und/oder aromatisch gebundenen Isocyanatgruppen der allgemeinen Formel (II) und
c) ≤ 12 Gew.-%, bevorzugt ≤ 10 Gew.-% an Verbindungen der allgemeinen Formel (III) 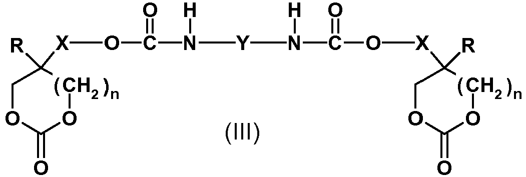 enthält,
wobei sich der Anteil an a) und c) jeweils auf die Gesamtmasse der Verbindungen a) und c) bezieht, der Anteil an b) sich auf die Gesamtmasse der Zusammensetzung A) bezieht,
und wobei
R für Wasserstoff oder einen gesättigten oder ungesättigten, linearen oder verzweigten, aliphatischen Rest mit 1 bis 7 Kohlenstoffatomen steht,
X für einen linearen oder verzweigten organischen Rest mit 1 bis 36 Kohlenstoffatomen steht, der gegebenenfalls Ether-, Ester- und/oder Carbonatgruppen enthalten kann,
n für 0 oder 1 steht und
Y für einen linearen oder verzweigten, aliphatischen oder cycloaliphatischen Rest mit 4 bis 18 Kohlenstoffatomen oder einen gegebenenfalls substituierten aromatischen oder araliphatischen Rest mit 6 bis 18 Kohlenstoffatomen steht.

4. Verfahren gemäß einem der Ansprüche 1 bis 3, **dadurch gekennzeichnet, dass** für die Formeln (I) und (II) der Komponenten a) und b) oder für die Formeln (I), (II) und (III) der Komponenten a), b) und c) gilt, dass
Y für einen linearen oder verzweigten, aliphatischen oder cycloaliphatischen Rest mit 6 bis 13 Kohlenstoffatomen steht.

5. Verfahren gemäß einem der Ansprüche 1 bis 4, **dadurch gekennzeichnet, dass** für die Formel (I) der Komponente a) oder die Formeln (I) und (III) der Komponenten a) und c) gilt, dass
R für Wasserstoff oder einen gesättigten linearen aliphatischen Rest mit 1 oder 2 Kohlenstoffatomen steht,
X für einen linearen oder verzweigten organischen Rest mit 1 bis 18 Kohlenstoffatomen steht, der gegebenenfalls Ether-, Ester- und/oder Carbonatgruppen enthalten kann, und
n für 0 oder 1 steht.

6. Verfahren gemäß einem der Ansprüche 1 bis 5, **dadurch gekennzeichnet, dass** für die Formel (I) der Komponente a) oder die Formeln (I) und (III) der Komponenten a) und c) gilt, dass
R für Wasserstoff oder einen gesättigten linearen aliphatischen Rest mit 1 oder 2 Kohlenstoffatomen steht,
X für eine Methylengruppe (-CH₂-) steht und
n für 0 oder 1 steht.

7. Verfahren gemäß einem der Ansprüche 1 bis 6, **dadurch gekennzeichnet, dass** die Polyolkomponente B) eine mittlere Funktionalität von 2 bis 6, bevorzugt von 2 bis 4 aufweist.

8. Verfahren gemäß einem der Ansprüche 1 bis 7, **dadurch gekennzeichnet, dass** als Komponente B) mehrwertige Alkohole, Ether- oder Esteralkohole und/oder polymere Polyole zum Einsatz kommen, wobei die polymeren Polyole ein zahlenmittleren Molekulargewicht Mₙ von 200 bis 22000 g/mol, bevorzugt 250 bis 18000 g/mol aufweisen.

9. Verfahren gemäß Anspruch 8, **dadurch gekennzeichnet, dass** als Komponente B) mehrwertige Alkohole und/oder Ether- oder Esteralkohole eingesetzt werden, die 2 bis 14 Kohlenstoffatome, bevorzugt 4 bis 10 Kohlenstoffatome enthalten.

10. Verfahren gemäß Anspruch 8, **dadurch gekennzeichnet, dass** als Komponente B) Polyether-, Polyester-, Polycarbonat- und/oder Polyacrylatpolyole eingesetzt werden.

11. Cyclische Carbonatstrukturen enthaltende Polyurethane, erhältlich nach einem Verfahren gemäß einem der Ansprüche 1 bis 10.

12. Verwendung der cyclische Carbonatstrukturen enthaltenden Polyurethane gemäß Anspruch 11 als Ausgangskomponente bei der Herstellung von vernetzbaren Bindemitteln.

13. Verwendung der cyclische Carbonatstrukturen enthaltenden Polyurethane gemäß Anspruch 11 als Ausgangskomponente bei der Herstellung von vernetzbaren Lack-, Dichtstoff- oder Klebstoff rohstoffen.

14. Vernetzbare Bindemittel enthaltend cyclische Carbonatstrukturen enthaltenden Polyurethane gemäß Anspruch 11.

## Claims

1. Process for preparing polyurethanes containing cyclic carbonate structures, by reacting at least
A) a composition containing
a) compounds of the general formula (I) containing one or more isocyanate groups and cyclic carbonate structures 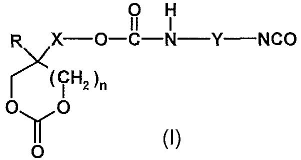 and
b) ≤ 1% by weight, based on the total mass of the composition A), of one or more monomeric diisocyanates having aliphatically, cyclo-aliphatically, araliphatically and/or aromatically bonded isocyanate groups of the general formula (II),
**OCN-Y-NCO** **(II)**
where
R is hydrogen or a saturated or unsaturated, linear or branched, aliphatic radical having 1 to 7 carbon atoms,
X is a linear or branched organic radical which has 1 to 36 carbon atoms and which may optionally contain ether, ester and/or carbonate groups,
n is 0 or 1 and
Y is a linear or branched, aliphatic or cycloaliphatic radical having 4 to 18 carbon atoms or an optionally substituted aromatic or araliphatic radical having 6 to 18 carbon atoms,
with
B) an at least difunctional polyol having a number-average molecular weight Mₙ of 62 to 22 000 g/mol, preferably 90 to 12000 g/mol,
while maintaining a ratio of equivalents of isocyanate groups to hydroxyl groups of 0.3:1 to 1.2:1.

2. Process for preparing polyurethanes containing cyclic carbonate structures according to Claim 1, **characterized in that** composition A) additionally contains compounds (c) of the general formula (III) 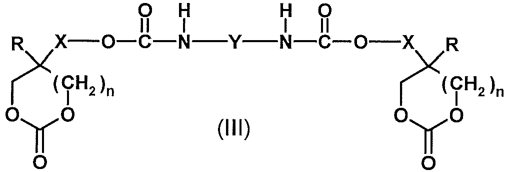 in an amount of 0.5% to 12% by weight, preferably 0.5% to 10% by weight, based on the total mass of components a) and c),
where
R is hydrogen or a saturated or unsaturated, linear or branched, aliphatic radical having 1 to 7 carbon atoms,
X is a linear or branched organic radical which has 1 to 36 carbon atoms and which may optionally contain ether, ester and/or carbonate groups,
n is 0 or 1 and
Y is a linear or branched, aliphatic or cycloaliphatic radical having 4 to 18 carbon atoms or an optionally substituted aromatic or araliphatic radical having 6 to 18 carbon atoms.

3. Process for preparing polyurethanes containing cyclic carbonate structures according to Claim 1, **characterized in that** composition A) contains
a) ≥ 88% by weight, preferably ≥ 90% by weight, of compounds of the general formula (I),
b) ≤ 1% by weight of one or more monomeric diisocyanates having aliphatically, cyclo-aliphatically, araliphatically and/or aromatically bonded isocyanate groups of the general formula (II) and
c) ≤ 12% by weight, preferably ≤ 10% by weight, of compounds of the general formula (III), 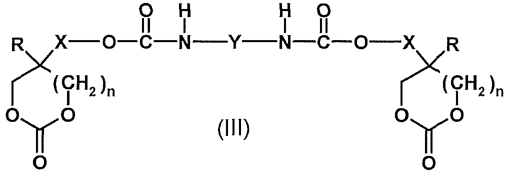 where the proportion of each of a) and c) relates to the total mass of the compounds a) and c), and the proportion of b) relates to the total mass of the composition A),
and where
R is hydrogen or a saturated or unsaturated, linear or branched, aliphatic radical having 1 to 7 carbon atoms,
X is a linear or branched organic radical which has 1 to 36 carbon atoms and which may optionally contain ether, ester and/or carbonate groups,
n is 0 or 1 and
Y is a linear or branched, aliphatic or cycloaliphatic radical having 4 to 18 carbon atoms or an optionally substituted aromatic or araliphatic radical having 6 to 18 carbon atoms.

4. Process according to any of Claims 1 to 3, **characterized in that**, for the formulae (I) and (II) of components a) and b), or for the formulae (I), (II) and (III) of components a), b) and c),
Y is a linear or branched, aliphatic or cycloaliphatic radical having 6 to 13 carbon atoms.

5. Process according to any of Claims 1 to 4, **characterized in that**, for the formula (I) of component a) or the formulae (I) and (III) of components a) and c),
R is hydrogen or a saturated linear aliphatic radical having 1 or 2 carbon atoms,
X is a linear or branched organic radical which has 1 to 18 carbon atoms and which may optionally contain ether, ester and/or carbonate groups, and
n is 0 or 1.

6. Process according to any of Claims 1 to 5, **characterized in that**, for the formula (I) of component a) or the formulae (I) and (III) of components a) and c),
R is hydrogen or a saturated linear aliphatic radical having 1 or 2 carbon atoms,
X is a methylene group (-CH₂-) and
n is 0 or 1.

7. Process according to any of Claims 1 to 6, **characterized in that** the polyol component B) has a mean functionality of 2 to 6, preferably of 2 to 4.

8. Process according to any of Claims 1 to 7, **characterized in that** components B) used are polyhydric alcohols, ether alcohols or ester alcohols and/or polymeric polyols, said polymeric polyols having a number-average molecular weight Mₙ of 200 to 22 000 g/mol, preferably 250 to 18 000 g/mol.

9. Process according to Claim 8, **characterized in that** components B) used are polyhydric alcohols and/or ether alcohols or ester alcohols containing 2 to 14 carbon atoms, preferably 4 to 10 carbon atoms.

10. Process according to Claim 8, **characterized in that** components B) used are polyether polyols, polyester polyols, polycarbonate polyols and/or polyacrylate polyols.

11. Polyurethanes containing cyclic carbonate structures, obtainable by a process according to any of Claims 1 to 10.

12. Use of the polyurethanes containing cyclic carbonate structures according to Claim 11 as a starting component in the production of crosslinkable binders.

13. Use of the polyurethanes containing cyclic carbonate structures according to Claim 11 as a starting component in the production of crosslinkable raw materials for varnishes, sealants or adhesives.

14. Crosslinkable binders comprising polyurethanes containing cyclic carbonate structures according to Claim 11.

## Revendications

1. Procédé de fabrication de polyuréthanes contenant des structures carbonate cycliques, par mise en réaction d'au moins
A) une composition contenant
a) un ou plusieurs composés de formule générale (I) contenant des groupes isocyanate et des structures carbonate cycliques 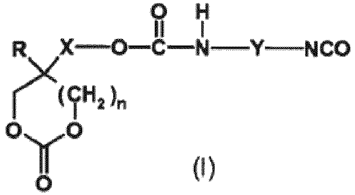 et
b) ≤ 1 % en poids, par rapport à la masse totale de la composition A), d'un ou de plusieurs diisocyanates monomères contenant des groupes isocyanate reliés aliphatiquement, cycloaliphatiquement, araliphatiquement et/ou aromatiquement, de formule générale (II)
**OCN-Y-NCO** **(II)**
R représentant l'hydrogène ou un radical aliphatique saturé ou insaturé, linéaire ou ramifié, de 1 à 7 atomes de carbone,
X représentant un radical organique linéaire ou ramifié de 1 à 36 atomes de carbone, qui peut éventuellement contenir des groupes éther, ester et/ou carbonate,
n représentant 0 ou 1, et
Y représentant un radical aliphatique ou cycloaliphatique, linéaire ou ramifié, de 4 à 18 atomes de carbone, ou un radical aromatique ou araliphatique éventuellement substitué de 6 à 18 atomes de carbone, avec
B) un polyol au moins bifonctionnel ayant un poids moléculaire moyen en nombre Mₙ de 62 à 22 000 g/mol, de préférence de 90 à 12 000 g/mol,
en maintenant un rapport équivalent entre les groupes isocyanate et les groupes hydroxyle de 0,3:1 à 1,2:1.

2. Procédé de fabrication de polyuréthanes contenant des structures carbonate cycliques selon la revendication 1, **caractérisé en ce que** la composition A) contient en outre des composés (c) de formule générale (III) 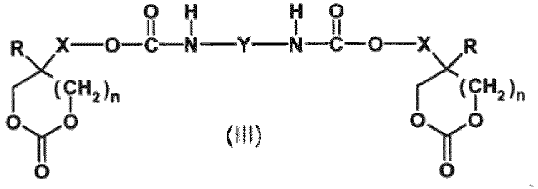 en une quantité de 0,5 à 12 % en poids, de préférence de 0,5 à 10 % en poids, par rapport à la masse totale des composants a) et c),
R représentant l'hydrogène ou un radical aliphatique saturé ou insaturé, linéaire ou ramifié, de 1 à 7 atomes de carbone,
X représentant un radical organique linéaire ou ramifié de 1 à 36 atomes de carbone, qui peut éventuellement contenir des groupes éther, ester et/ou carbonate,
n représentant 0 ou 1, et
Y représentant un radical aliphatique ou cycloaliphatique, linéaire ou ramifié, de 4 à 18 atomes de carbone, ou un radical aromatique ou araliphatique éventuellement substitué de 6 à 18 atomes de carbone.

3. Procédé de fabrication de polyuréthanes contenant des structures carbonate cycliques selon la revendication 1, **caractérisé en ce que** la composition A) contient
a) ≥ 88 % en poids, de préférence ≥ 90 % en poids, de composés de formule générale (I),
b) ≤ 1 % en poids d'un ou de plusieurs diisocyanates contenant des groupes isocyanate reliés aliphatiquement, cycloaliphatiquement, araliphatiquement et/ou aromatiquement, de formule générale (II), et
c) ≤ 12 % en poids, de préférence ≤ 10 % en poids, de composés de formule générale (III) 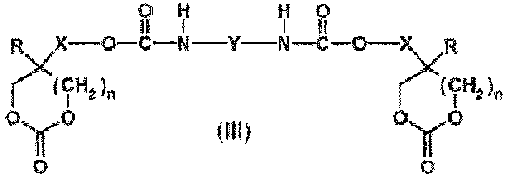 la proportion de a) et c) se rapportant à chaque fois à la masse totale des composés a) et c), la proportion de b) se rapportant à la masse totale de la composition A),
et
R représentant l'hydrogène ou un radical aliphatique saturé ou insaturé, linéaire ou ramifié, de 1 à 7 atomes de carbone,
X représentant un radical organique linéaire ou ramifié de 1 à 36 atomes de carbone, qui peut éventuellement contenir des groupes éther, ester et/ou carbonate,
n représentant 0 ou 1, et
Y représentant un radical aliphatique ou cycloaliphatique, linéaire ou ramifié, de 4 à 18 atomes de carbone, ou un radical aromatique ou araliphatique éventuellement substitué de 6 à 18 atomes de carbone.

4. Procédé selon l'une quelconque des revendications 1 à 3, **caractérisé en ce que**, pour les formules (I) et (II) des composants a) et b) ou pour les formules (I), (II) et (III) des composants a), b) et c), Y représente un radical aliphatique ou cycloaliphatique linéaire ou ramifié de 6 à 13 atomes de carbone.

5. Procédé selon l'une quelconque des revendications 1 à 4, **caractérisé en ce que**, pour la formule (I) du composant a) ou les formules (I) et (III) des composants a) et c),
R représente l'hydrogène ou un radical aliphatique linéaire saturé de 1 ou 2 atomes de carbone,
X représente un radical organique linéaire ou ramifié de 1 à 18 atomes de carbone, qui peut éventuellement contenir des groupes éther, ester et/ou carbonate, et
n représente 0 ou 1.

6. Procédé selon l'une quelconque des revendications 1 à 5, **caractérisé en ce que**, pour la formule (I) du composant a) ou les formules (I) et (III) des composants a) et c),
R représente l'hydrogène ou un radical aliphatique linéaire saturé de 1 ou 2 atomes de carbone,
X représente un groupe méthylène (-CH₂-) et
n représente 0 ou 1.

7. Procédé selon l'une quelconque des revendications 1 à 6, **caractérisé en ce que** le composant polyol B) présente une fonctionnalité moyenne de 2 à 6, de préférence de 2 à 4.

8. Procédé selon l'une quelconque des revendications 1 à 7, **caractérisé en ce que** des alcools, éther- ou ester-alcools polyvalents et/ou des polyols polymères sont utilisés en tant que composant B), les polyols polymères présentant un poids moléculaire moyen en nombre Mₙ de 200 à 22 000 g/mol, de préférence de 250 à 18 000 g/mol.

9. Procédé selon la revendication 8, **caractérisé en ce que** des alcools et/ou éther- ou ester-alcools polyvalents sont utilisés en tant que composant B), qui contiennent 2 à 14 atomes de carbone, de préférence 4 à 10 atomes de carbone.

10. Procédé selon la revendication 8, **caractérisé en ce que** des polyéther-, polyester-, polycarbonate- et/ou polyacrylate-polyols sont utilisés en tant que composant B).

11. Polyuréthanes contenant des structures carbonate cycliques, pouvant être obtenus par un procédé selon l'une quelconque des revendications 1 à 10.

12. Utilisation des polyuréthanes contenant des structures carbonate cycliques selon la revendication 11 en tant que composant de départ pour la fabrication de liant réticulables.

13. Utilisation des polyuréthanes contenant des structures carbonate cycliques selon la revendication 11 en tant que composant de départ pour la fabrication de matières premières de vernis, d'agents d'étanchéité ou d'adhésifs réticulables.

14. Liants réticulables contenant des polyuréthanes contenant des structures carbonate cycliques selon la revendication 11.
